(19) Europäisches Patentamt
European Patent Office
Office européen des brevets

(11) Publication number: **0 463 571 A2**

(12) # EUROPEAN PATENT APPLICATION

(21) Application number: **91110173.1**

(22) Date of filing: **20.06.91**

(51) Int. Cl.$^5$: **C12N 15/62**, C12N 15/70, C12N 15/12, C12P 21/02, C12N 1/21, //(C12N1/21, C12R1:19)

The microorganisms have been deposited with Ferm under numbers BP-2950 and BP-3171.

(30) Priority: **20.06.90 JP 159909/90**
**30.11.90 JP 330146/90**

(43) Date of publication of application:
**02.01.92 Bulletin 92/01**

(84) Designated Contracting States:
**AT BE CH DE FR GB IT LI NL SE**

(71) Applicant: **TEIJIN LIMITED**
**6-7, Minamihommachi 1-chome Chuo-ku**
**Osaka(JP)**

(72) Inventor: **Eguchi, Hiroshi**
**Haitsu Obama 104, 3-2-5, Tamadaira**
**Hino-shi, Tokyo(JP)**
Inventor: **Kamimura, Takashi**
**7555 Charmant St. Apt. Nr. 1114**
**San Diego, CA 92122(US)**
Inventor: **Sugiyama, Takashi**
**163-2, Shouei-machi, Kasamatsu-cho**
**Hatori-gun, Gifu-ken(JP)**
Inventor: **Hosoda, Kenji**
**Hachiou Haitsu Sennin-cho 306, 4-7-26,**
**Sennin-cho**
**Hachioji-shi, Tokyo(JP)**

(74) Representative: **Kraus, Walter, Dr. et al**
**Patentanwälte Kraus, Weisert & Partner**
**Thomas-Wimmer-Ring 15**
**W-8000 München 22(DE)**

(54) **Recombinant human osteocalcin.**

(57) This invention relates to recombinant human osteocalcin. Particularly, this invention relates to a series of techniques for expressing human osteocalcin using bacterial cells. More specifically, this invention relates to a DNA sequence for the expression of human osteocalcin, a vector for the expression of human osteocalcin, a transformant wherein the vector is introduced, and a process for preparing human osteocalcin or a protein equal thereto using the transformant.

EP 0 463 571 A2

## Detailed Description of the Invention

⟨Industrially Applicable Field⟩

This invention relates to recombinant human osteocalcin. Particularly, this invention relates to a series of techniques for expressing human osteocalcin using bacterial cells. More specifically, this invention relates to a DNA sequence for the expression of human osteocalcin, a vector for the expression of human osteocalcin, a transformant wherein the vector is introduced, and a process for preparing human osteocalcin or a protein equal thereto using the transformant.

⟨Prior Art and Problems to be Solved by the Invention⟩

Osteocalcin is one of the Gla proteins found in the bone matrix and a protein capable of binding to bone calcium depending on vitamin K. Human osteocalcin has a molecular weight of 5800 and is constituted by 49 amino acids (J. W. Poster et al., J. Biol. Chem. 255, 8685 to 8691, 1980). This human osteocalcin has the amino acid sequence shown in Figure 1. The amino acid sequence in Figure 1 denotes the amino acid sequence wherein none of the Glu residues are carboxylated at the $\gamma$-position, namely converted to Gla residues.

Osteocalcin has Gla residues ($\gamma$-carboxyglutamic acid residues) and has strong affinity to hydroxyapatite, and is thus surmised to have an important role in the formation of bone matrix.

It is necessary for human osteocalcin to be provided in a large amount for the purpose of further studying the clinical significance of human osteocalcin or researching and developing the possibility as a drug.

However, human osteocalcin can presently be obtained only from human bones through high-degree purification, and it is extremely difficult to get it not only in a large amount but even in a small amount.

As one method to solve this problem, it is contemplated to apply gene engineering techniques. For example, it is reported that the fusion expression of the human osteocalcin gene was tried [N. OYAMA et al., Previous Lectures for the 11th Nippon Bunshiseibutsu Gakkai (Japan Molecular Biology Society), page 161, 3D-04 (1988)].

This report is made under the title of "Expression of the Human Osteocalcin Precursor Gene in Escherichia coli", and extremely simple in contents. According to the report, a fused gene is prepared wherein the human osteocalcin gene was ligated tandem by 4 copies into the downstream of the pstS gene derived from Escherichia coli and a DNA sequence encoding the lys (lysine) residue was inserted between the respective genes, and fusion expression is carried out. However, in this report, what was done is only that its expression was ascertained by Western blotting using a monoclonal antibody recognizing the C-terminus of bovine osteocalcin, and there are not performed the purification of the fused gene product and the monomerization by lysyl endopeptidase.

Further, even if a protein is obtained as the gene product by the above method, it will be human osteocalcin having no physiological activity, i.e. human osteocalcin wherein none of the Glu residues in the amino acid sequence are converted to Gla residues (hereinafter referred to as human osteocalcin wherein Glu to Gla conversion is not made), and it cannot be believed that it is possible to obtain by this method human osteocalcin wherein all or part of the Glu residues in the amino acid sequence are converted to Gla residues (hereinafter referred to as human osteocalcin wherein Glu to Gla conversion is made) and which thus has a physiological activity.

Thus, it is desired to provide in a large amount human osteocalcin having a physiological activity.

On the other hand, proteins containing Gla residues include besides human osteocalcin, fibrinolysis and coagulation proteins such as prothrombin, factor IX, factor VII, protein C, protein S and protein Z. It is known that in these proteins the Glu residues are converted to Gla groups in the living body by the action of $\gamma$-carboxylase (vitamin K-dependent carboxylase) [J. W. Suttie et al., J. Biol. Chem. 251, 5827-5830 (1970)].

Further, reports were made as follows that in case of factor IX, protein C, prothrombin, etc. a propeptide is necessary for $\gamma$-carboxylase to operate effectively.

(1) L. C. Pan et al, P.N.A.S., 82 6109-6113 (1985).
(2) J. W. Sutlie et al., P.N.A.S., 84 634-637 (1987)
(3) B. E. Furio et al., WO88-3926
(4) D. C. Foster et al., Biochemistry 26 7003-7011 (1987)

However, findings on a propeptide for Glu to Gla conversion on osteocalcin and its action have never been so far obtained.

Thus, the present inventors prosecuted their studies about a series of techniques for obtaining physiologically active human osteocalcin in a large amount utilizing bacterial cells. Namely, they prosecuted their studies for the purpose of the provision of a DNA sequence making it possible that a protein containing human osteocalcin is effectively expressed in bacterial cells, and human osteocalcin is monomerized from the obtained protein and converted to human osteocalcin wherein all or part of the Glu residues in the amino acid sequence are converted to Gla residues; a plasmid containing the DNA sequence; cells transformed by the plasmid; and the expression and purification of human osteocalcin.

⟨Means for Solving the Problems⟩

As a result of intensive studies to attain the above object of the invention, the present inventors have reached the invention.

According to this invention is provided first a DNA sequence for the expression of human osteocalcin (hereinafter referred to as a human osteocalcin-expressing DNA sequence) represented by the following formula [I]

A-B-C-D [I]

wherein,

D represents a DNA sequence encoding human osteocalcin or a protein physiologically equal thereto (sequence D),

C represents a DNA sequence encoding a cleavage sequence peptide (sequence C),

B represents B-1 and/or B-2 wherein B-1 represents DNA sequence encoding an additional polypeptide for expressing human osteocalcin in a large amount in host cells (sequence B-1) and B-2 represents a DNA sequence encoding a polypeptide recognized by an enzyme capable of Glu to Gla conversion on human osteocalcin (sequence B-2), and

A represents a DNA sequence which may optionally be present and encodes a signal peptide (sequence A).

Human osteocalcin having a physiological activity can be obtained by, according to the later described method, preparing a plasmid containing a human osteocalcin-expressing DNA sequence represented by the formula [I] of the invention, transforming host cells with the obtained plasmid, and culturing the transformed host cells.

Detailed description is made below about the human osteocalcin-expressing DNA sequence represented by the formula [I] of the invention, a plasmid containing the same, the transformation of host cells with this plasmid, and the expression, separation, purification and ascertainment of human osteocalcin.

⟨Brief Description of the Drawings⟩

Figure 1 denotes a DNA sequence as one example of the DNA sequence encoding human osteocalcin or the protein equal thereto of the invention (sequence D).

Figure 2 denotes a DNA sequence as one embodiment of the DNA sequence encoding the peptide for the expression of human osteocalcin or the protein physiologically equal thereto (sequence B-2).

Figure 3 denotes a DNA sequence as one example of the DNA sequence encoding the cleavage sequence peptide of the invention (sequence C).

Figure 4 denotes a DNA sequence as one example of the DNA sequence encoding the signal peptide of the invention (sequence A).

Figure 5 denotes the amino acid sequence of human osteocalcin.

Figure 6 denotes the human osteocalcin-expressing DNA sequence of the invention in Example 1. In the figure, the solid lines denote the boundaries of the synthesized DNA fragments and the dotted lines denote cleavage faces with the restriction enzymes. Further, figures ① to ⑫ denote respectively the oligonucleotides synthesized in Example 1 when the above DNA sequence is supposed to be divided at the boundary positions of the synthesized DNA fragments.

Figure 7 denotes the human osteocalcin-expressing DNA sequence in Example 1. In the figure, sequence A, sequence B, sequence C and sequence D denote a signal peptide, the part recognized by an enzyme capable of Glu to Gla conversion on human osteocalcin, a cleavage sequence and the sequence part encoding human osteocalcin, respectively.

Figure 8 denotes a process of preparing a modified plasmid pBPR9 of cloning vector pHSG399 in Example 1.

Figure 9 denotes a process of the construction of plasmid pBPR-PRE in Example 1.

Figure 10 denotes a process of the construction of plasmid pBPR-MOC in Example 1.

Figure 11 denotes a process of the construction of plasmid pBPR-OC in Example 2.

Figure 12 denotes a process of the construction of plasmid pKK-OC in Example 3.

Figure 13 denotes the synthesized fragments prepared in Example 6.

Figure 14 denotes a process of the construction of plasmid pOC28 in Example 6.

Figure 15 denotes a process of the construction of plasmid pKOC 28 in Example 6.

Figure 16 denotes the results by human osteocalcin EIA (Engyme Immuno Assay) in Example 8.

Figure 17 denotes the results of the measurement of the concentrations of the human osteocalcin-containing polypeptide in Example 9.

Figure 18 denotes peptides used in the preparation of the antibodies in Reference examples 1 to 3. In the figure, (a), (b) and (c) denote peptides Ost-N(20) in Reference example 1, Ost-C(7) in Reference example 2 and Ost-C(15) in Reference example 3, respectively.

Figure 19 denotes the results of the evaluation on activity of the beads immobilizing the antibody in Reference example 4.

Figure 20 denotes a calibration curve on the intact human osteocalcin measurement system in Reference example 4.

Figure 21 denotes a design for the construction of the DNA sequence for the expression of the human growth hormore-human osteocalcin fused protein in Example 10.

Figure 22 denotes the design of the Linker DNA portion in Example 10.

Figure 23 denotes a process of the construction of plasmid pGH-POCI in Example 11.

Figure 24 denotes a process of the construction of plasmid pGH-POCII in Example 11.

Figure 25 denotes the results of SDS-PAGE in Example 12, and (A) and (B) denote the results of plasmid pGH-POCI and a plasmid pGH-POCII, respectively.

Figure 26 denotes the results of SDS-PAGE in Example 14.

In the human osteocalcin-expressing DNA sequence of the formula [I] of the invention, sequence D represents a DNA sequence encoding human osteocalcin or a protein physiologically equal thereto. As such a DNA sequence, there can for example be mentioned the DNA sequence shown in Figure 1. In Figure 1 the amino acid sequence corresponding to the DNA sequence is shown in the parenthesis.

The protein physiologically equal to such human osteocalcin means

(i) a modified body of human osteocalcin which either has, as it is, physiological activities similar to those of osteocalcin, or does not have physiological activities similar thereto but has physiological activities similar to human osteocalcin when all or part of the Glu residues in the amino acid sequence were converted Gla residues, or

(ii) a protein detectable by a method of immunologically assaying intact human osteocalcin.

In this connection, the modified body of human osteocalcin includes, for example in the sequence of human osteocalcin consisting of 49 amino acids shown in Figure 1, one wherein part of the amino acids, preferably 1 to 5 amino acids are replaced by other amino acids, one wherein 1 to 5 amino acids are deleted, one wherein 1 to 5 other amino acids are inserted, or one in combination thereof.

Further as the above method of immunologically assaying intact human osteocalcin, any method can be used so long as it is capable of immunologically assaying and detecting intact human osteocalcin, in distinction from its fragments, in a human osteocalcin-containing sample, and there can, for example, be mentioned the method which was previously proposed by the present inventors and disclosed in the PCT/JP90/00155 application (Application date in Japan: February 10, 1989; Application number: 30,003/1989). This enzyme immunoassay for intact human osteocalcin proposed by the present inventors is described in the later Reference examples 1 to 4.

Sequence C in the above formula [I] represents a DNA sequence encoding a cleavage peptide. Examples of sequences usable as such DNA sequences and amino acid sequence cleavable with the propeptidase of human osteocalcin, an amino acid sequence cleavable with thrombin, an amino acid sequence cleavable with factor Xa (FXa), an amino acid sequence cleavable by the CNBr method (Met), an amino acid sequence cleavable with lysyl endopeptidase (Lys), etc., respectively. Preferred among them is the sequence cleavable with thrombin, for example the DNA sequence shown in Figure 3. The parenthesis in Figure 3 denotes the amino acid sequence corresponding to the DNA sequence.

Further, sequence B represents a DNA sequence encoding a peptide necessary for expressing human osteocalcin or a protein physiologically equal thereto. Sequence B consists of sequence B-1 and/or sequence B-2. Sequence B-1 is a DNA sequence encoding an additional polypeptide for expressing human osteocalcin in a large amount in host cells, and it is preferred that sequence B-1 either stably exist in the host cells or is a DNA sequence encoding a polypeptide forming an inclusion body. It is preferred to select sequence B-1 depending on the kind of the host cells. For example, when Escherichia coli is used as host cells, specific examples of sequence B-1 are DNA sequences encoding β-galactosidase, β-lactamase, chloramphenicol acetyltransferase, alkaline phosphatase, Staphylococcus protein A, β interferon, $\alpha_1$ antitrypsin, human growth hormone, etc., respectively. Particularly preferred is a DNA sequence encoding a human growth hormone-derived polypeptide.

On the other hand, sequence B-2 is a DNA sequence encoding a polypeptide recongnized by the enzyme capable of Glu to Gla conversion on human osteocalcin. As a sequence usable as such a DNA sequence, there can be mentioned a DNA sequence encoding either a propeptide derived from a Gla protein necessary for the enzyme capable of Glu to Gla conversion to act effectively or its derivative (for example, one wherein 1 to 5 amino acid residues of the propeptide are deleted, inserted on replaced) (sequence B-2). Preferred among such DNA sequences are DNA sequences encoding the propeptide of human osteocalcin and the propeptide of prothrombin, respectively. As such a DNA sequence, there can for example be mentioned the DNA sequence shown in Figure 2 and encoding the propeptide of human osteocalcin. The parenthesis in Figure 2 shows the amino acid sequence corresponding to the DNA sequence.

Although B in the formula [I] of the invention can be any of sequence B-1 and sequence B-2, a DNA sequence is preferred wherein sequence B-1 and sequence B-2 are combined.

In this case, sequence B-1 and sequence B-2 are preferably arranged in this order, and further in this instance a sequence is also included having a structure such that sequence B-1 and sequence B-2 are ligated with a DNA sequence encoding a cleavage peptide as denoted in the above sequence C.

In this invention, such sequences D, B and C can be arranged in the order of B-C-D represented in the formula [I] to give a human osteocalcin-expressing DNA sequence.

Further, in the DNA sequence of the formula [I] of the invention, it is possible to ligate to the N-terminus of the B-C-D sequence a DNA sequence encoding a signal peptide (sequence A). Sequence A can optionally be ligated according to necessity and an embodiment wherein sequence A is not ligated is included in the invention, too. All necessitated about sequence A is that it is a DNA sequence encoding a signal peptide which can be secreted in the host cells, and a proper sequence is selected depending on the kind of host cells. When Escherichia coli is adopted as host cells, there can be used as sequence A a DNA sequence encoding a signal peptide such as Omp A, Pho A, Omp F, protein A, β-lactamase (penicillinase), endotoxin or human growth hormone. The singnal peptide of (prepeptide) of human osteocalcin has a charge at the N-terminus of +1 (arginine residue). On the other hand in the signal peptide of prolipoprotein or the like, there is a report that when the charge of the region of the N-terminus is +2, most sufficient secretion takes place (Inouye, S. et al., P.N.A.S., 79, 3438-3441, 1982).

As the signal peptide of the invention is preferred among those above-mentioned the signal peptide of human osteocalcin or a modified body thereof wherein the charge at the N-terminus is changed to +2. As such an A sequence there can for example be mentioned the DNA sequence shown in Figure 4. The parenthesis in Figure 4 denotes the amino acid sequence corresponding to the DNA sequence.

Further, this invention provides a vector for the expression of human osteocalcin for expressing the human osteocalcin-expressing DNA sequence represented by the formula [I] in host cells, and recombinant host cells wherein transformation by the vector was made.

Although the expression of human osteocalcin becomes possible by obtaining a vector capable of expression in host cells from the human osteocalcin-expressing DNA sequence (a human osteocalcin-expressing vector) and using recombinant host cells wherein transformation was made with this vector, it is desirable in this case to provide a DNA sequence having a function to regulate the expression of human osteocalcin (an expression regulating sequence). As such sequences, there can be mentioned sequences having a function of promoting or in some case inhibiting the expression, for example promoters, enhancers, etc., and preferred among them are expression regulating sequences capable of functioning in Escherichia coli, for example tryptophan operon promoter, tac promoter, etc.

The sequence B-C-D or A-B-C-D in the formula [I] of the invention can be changed into an intracellular expression type gene by ligating the sequence into the downstream of a proper promoter and SD (Shine-Dalgarno) sequence.

As usable promoters, there can be mentioned tryptophan operon promoter (trp promoter), lactose operon promoter (lac promoter), tac promoter, PL promoter lpp promoter, etc, and preferred among them are trp promoter and lac promoter.

For the effective expression of human osteocalcin as the objective of the invention, it is preferred to ligate a promoter, SD sequence, a translation initiation codon, the DNA sequence of the formula [I], a translation termination codon and a terminator (transcription termination sequence) in this order.

A human osteocalcin expressing plasmid can be prepared by inserting the thus ligated sequence into a proper expression vector, for example a plasmid.

Usable as plasmid vectors for the expression in Escherichia coli are pUCl8, pHSG398, pRIT2T, pUEXl to 3, pKK223-3, pINIIIAl, pTTQl8, pGEMEX-l, pHG-L9, pKK233-2, etc.

In the invention, as a human osteocalcin-expressing plasmid is preferred pkk233-2 wherein a sequence containing tac promoter, SD sequence, a translation initiation codon, the cloning site and a terminator in this order is inserted into a proper cloning site. Particularly preferred as an expression plasmid is pGH-L9 having trp promoter, SD sequence, a translation initiation codon, a DNA sequence encoding human growth hormone, and a terminator in this order.

Particularly in case of the latter expression plasmid, by constructing a sequence B-C-D as a human osteocalcin-expressing DNA sequence containing the DNA sequence part encoding human growth hormone in the expression plasmid, a human osteocalcin-expressing plasmid is obtained containing trp promoter, SD sequence, a translation initiation codon, Sequence B-C-D, and a terminator in this order.

Transformed host cells capable of producing human osteocalcin can be obtained by introducing the expression plasmid into the corresponding host cells.

As such host cells to be transformed, there can be mentioned microorganisms, animal tissue culture cell strains, animal cells, but there is no particular limitation thereon so long as they are host cells suitable for expression of human osteocalcin. However, microorganisms, particularly Escherichia coli is preferably used. When Escherichia coli is used as host cells, there can be used HBl0l, DHl, C600, SCSl, JMl09, XLl-Blue or the like, all of which are derived from the Escherichia coli K12 strain, and preferred among them is HBl0l, and also preferred is the XLl-BlueF$^-$strain wherein F factor (which contains the lac I repressor gene) of XLl-l-Blu XLl-Blue is removed.

Further, according to this invention is provided a process for preparing human osteocalcin or a protein physiologically equal thereto which comprises culturing the thus transformed host cells to produce in the culture broth a polypeptide-containing protein represented by the following formula [II]

B'-C'-D'      [II]

wherein,

- D' represents human osteocalcin or the protein physiologically equal thereto,
- C' represents a cleavage sequence peptide, and
- B' represents B'-1 and/or B'-2 wherein B'-1 represents a polypeptide recognized by an enzyme capable of Glu to Gla conversion on human osteocalcin and B'-2 represents an additional polypeptide for expressing human osteocalcin in a large amount in the host cells; and either (a)

cleaving this protein and isolating human osteocalcin or the protein physiologically equal thereto (D'), or (b) converting all or part of the Glu residues in the D' protein in the protein to Gla residues, cleaving the resulting protein and isolating human osteocalcin or the protein physiologically equal thereto (D').

Further, according to this invention is provided human osteocalcin or the protein physiologically equal thereto obtained by the above preparation process.

As the D' protein, B' peptide and C' peptide there can for example be mentioned amino acid sequences corresponding respectively to the DNA sequences represented by the sequences B, C and D, further specifically those shown in Figures 1 to 3.

In the preparation process of the invention, the culture of the recombinant host cells is carried out using preferably the ZxYT medium, M9CA medium, and by such culture is produced in the culture broth a protein containing a polypeptide represented by the formula [II]. In the above, the culture broth includes the inside of the cells and/or the medium depending on whether the host cells used for the culture extracellularly produce the objective protein extra cellularly.

In the invention, the D' protein part of the protein is then subjected to Glu to Gla conversion. For example, in case of host cells making intracellular secretion, the cells are collected preferably at the later logarithmic phase, the periplasmic fraction is obtained by a known method such as, for example, the osmotic shock method, and the objective protein is purified from the obtained periplasmic fraction by a known method such as, for example, the antibody affinity column method. This purified or unpurified objective protein is then treated in vitro with $\gamma$-carboxylase (for example one derived from the liver) to convert all or part of the Glu residues in the D' protein to Gla residues. The Glu to Gla-converted D' protein is then separated from the B' peptide by cleavage at the part of the C' peptide, and the D' protein is isolated. The isolation of the human osteocalcin wherein Glu to Gla conversion was made can be carried out by a known method, for example using a hydroxyapatite column or an antibody affinity column.

Further in the invention, it is possible to purify the protein obtained by the above process and convert all or part of the Glu residues of the D' protein part to Gla residues. For example, in case of host cells forming an inclusion body, the cells are collected preferably at the final logarithmic phase and destroyed by a known method, for example ultrasonication, the inclusion body is obtained as the precipitate by centrifugation and solubilized using a protein-denaturing agent such as, for example, urea, and then dialysis is carried out to obtain a crudely purified aqueous solution of the protein. The protein is further purified by a known method, for example reverse phase high performance liquid chromatography. The purified human osteocalcin-fused protein is then treated in vitro with $\gamma$-carboxylase (for example, one derived from the liver) to convert all or part of the Glu residues of the D' protein to Gla residues.

The Glu to Gla conversion can be carried out by a method known per se. Specifically, carboxylation is carried out by the method disclosed in J. E. Knobloch and J. W. Suttie, J. Biol. Chem., 262, 15334-15337 (1987); C. Vermeer, Japanese Laid-Open Patent Publication No. 502956/1988; or the like. For example, the mirosome fraction is prepared from the liver of a rat or cattle (J. A. sadowski et al., J. Biol. Chem., 251, 2770-2776 (1976)) and suspended in a solution having the composition of 0.25 M sucrose-0.025 M imidazole-0.5 M KCl (pH7.2) and 1.5 % Triton X-100. To this suspension are added a carbonate buffer of 2 mM NADPH, 1 mM DTT, 1 mM EDTA, 1 mM $MnCl_2$ and 0.01 to 1 mM B'-C'-D' peptide, and further 100 µg/ml vitamin $K_1H_2$ to start reaction. Incubation is carried out at 17 °C to conduct carboxylation. Thereafter, the resulting B'-C'-D' peptide wherein Glu to Gla conversion was made is purified with an affinity column using an antibody against the C-terminal 7 residue peptide (WO-9009587) and subjected to thrombin treatment to cleave the B'-C' peptide. The reaction mixture is further passed through an affinity column using an antibody against the N-terminal 20 residue peptide (WO-9009587) and then a hydroxyapatite column to obtain the D' peptide wherein Glu to Gla conversion was made (human osteocalcin).

Meanwhile, when the A-B-2-C-D sequence (wherein C is a series cleaved with, e.g., propeptidase is expressed in animal cells (e.g., CHO cells), the D' peptide converted to Gla is obtained.

The thus obtained human osteocalcin wherein Glu to Gla conversion was not made and human osteocalcin wherein Glu to Gla conversion was made can be utilized as a standard substance for enzyme immunoassay, as an antigen for the preparation of its antibody, in the purification of its antibody, and further as a drug in relation to bone metabolism.

〈Example〉

This invention is more specifically described below by examples, but the invention is not limited thereto. Various gene engineering techniques adopted in examples were conducted by the following methods.

(1) Cleavage of DNA with restriction enzyme (Method 1)

① Universal Buffer of Takara Shuzo Co., Ltd. was used for restriction enzymes EcoRI, Ban HI, XbaI, DraI, AvaIII (EcoT22I), XhoI, KpnI and PstI.
② 10X Buffer of Nippon Gene Co., Ltd. was used for Nhe I and Bgl I.
③ One-Phor-All Buffer PLUS of Pharmacia Co. was used for NcoI, PvuI and BclI.

(2) Agrose electrophoresis (Method 2)

After cleavage with the restriction enzyme, 3 µl of an aqueous solution of 0.25 % Bromophenol Blue and 50 % glycerol was added, and agarose gel electrophoresis (gel concentration 0.8 to 2 %) was carried out.

An aqueous solution of 40 mM Tris acetate-1 mM EDTA was used as en electrophoresis buffer.

(3) Recovery of DNA fragments (> several hundred bp) from agarose gel (Method 3)

Agarose electrophoresis was conducted using low melting agarose (produced by Sigma Co.), a band corresponding to the objective DNA fragments was cut out and the DNA fragments were recovered with GENECLEAN® Kit (purchased from Funakoshi Co., Ltd. ).

(4) Recovery of DNA fragments (< several hundred bp) from agarose gel (Method 4)

Agarose electrophoresis was carried out for separation, a DEAE membrane (S & S Co., NA-45) was inserted immediately in front of the band corresponding to the objective DNA fragments, and agarose electrophoresis is carried out again to make the membrane adsorb the objective DNA fragments. The membrane was washed with NET Buffer (0.15 M NaCl-0.1 mM EDTA-20 mM Tris. HCl (pH8)) and then cut into pieces, High Sal + $NE^-$ Buffer (0.1M NaCl-0.1 mM EDTA-20 mM Tris.HCl (pH8)) was added, and incubation was carried out at 65°C for 1 hour to elute the objective DNA fragments, which were then recovered by ethanol precipitation.

(5) Ligation reaction with T4-DNA ligase (Method 5)

Ligation was conducted by carrying out reaction at 16°C for 3 hours or more using the DNA ligation kit (Takara Shuzo Co., Ltd.).

(6) Repair reaction (Blunting reaction of sticky end) (Method 6)

Repair reaction was carried out according to the manual of the DNA blunting kit (Takara Shuzo Co., Ltd.) using the kit.

(7) Preparation and transformation of competent cells (Method 7)

The transformation of Escherichia coli XLI-Blue strain was carried out according to a modified method of the usual $CaCl_2$ method (the method of M. V. Norgard et al.). Namely, the 18 hours culture of Escherichia coli XLI-Blue was inoculated in 5 ml of the LB medium (1 % trypton, 0.5 % yeast extract, 0.5 % NaCl, pH 7.2) and grown until the turbidity at 600 nm (OD600) of the culture broth containing the cells became 0.5 to 0.6.

The cells were collected by centrifuging 30 ml of the culture broth, washed with 15 ml of ice cooled 0.1M $MgCl_2$, collected again by centrifugation and suspended in 15 ml of 0.1M $CaCl_2$, and the suspension was allowed to stand in ice water for 20 minutes or more. The cells were collected again by centrifugation, and resuspended in 1.5 ml of 0.1M $CaCl_2$, the DNA solution after 2 ml ligation reaction (- 10 µl (- 1 µg)) was added to 0.2 ml of the suspension, followed by incubation at 0°C for 30 minutes or more. The mixture was then incubated at 42°C for 2 minutes, cooled again at 0°C, and incubated at 37°C for 1 hour with the addition of 0.8 ml of the LB medium. This culture was inoculated on an agar plate for selection (an LB agar medium containing 50 µg/ml ampicillin or 30 µg/ml chloramphenicol) and cultured at 37°C overnight to grow transformants.

Plasmid DNAs were prepared from the obtained drug resistant colonies using the method of Birnboim et al. (Birnboim, H.C. and J. Doky, Nucleic Acids Res., 7, 1513 (1979)), each hydrolyzed using a proper restriction enzyme and analyzed for its pattern by agarose electrophoresis to obtain an objective clone.

(8) Determination of DNA sequence (Method 8)

The determination was carried out according to the method of Chen (Chen, E. Y. and Seeburg, P.H., DNA, 4, 165 (1985)) using the plasmid DNA as a template, M13 primer M3, RV (Takara Shuzo Co., Ltd.) as a primer, and further Deaza T7 Sequencing Mixes (produced by Pharmacia Co.) and T7 Sequencing Kit (produced by Pharmacia Co.).

(9) Other methods

All the DNA manipulations were carried out by the method of Maniatis et al. (Molecular Clonings Cold Spring Harbor Laboratory, New York, 1982).

(10) Recovery of periplasmic fraction by the osmotic method (Method 10)

The culture broth was centrifuged to collect the cells. The sedimented cells (cell amount = ($OD_{600}$ value) x (culture broth (ml)) = 2 to 3) were suspended in 0.15 ml of the solution A (20 % sucrose, 10 mM Tris HCl (pH 7.6)) and incubated in ice water for 10 minutes. The suspension was centrifuged at 7,000 rpm and 4°C for 10 minutes, and the precipitated cells were resuspended in 0.15 ml of cooled distilled water and incubated in ice-water for 10 minutes. During the incubation the suspension was intensely stirred several times by vortex or the like (the outer membrane of the cells is bursts by osmotic shock and the proteins inside the periplasm come out). The mixture was then centrifuged again at 7,000 rpm and 4°C for 10 minutes, and the supernatant was recovered as a periplasm fraction.

The precipitate was suspended in 0.15 ml of 50 mM Tris HCl (pH 8) and fractured using an ultrasonic fracturing apparatus (Toso Co., Ltd., UCD-200T type). The fractured matter was centrifuged at 15,000 rpm and 4°C for 10 minutes, the supernatant was subjected to cytoplasmic fractionation, and the precipitate was obtained as the (outer membrane + inner membrane + inclusion body) fraction. The precipitate was suspended in 0.15 ml of 50 mM Tris HCl (pH 8) containing 1 % SDS 10 % glycerol and incubated at 95°C for 5 minutes to give a solution.

(11) Enzyme immunoassay of intact human osteocalcin

A method to prepare the antibody and the constitution of the assay system are described below.

Reference example 1: Preparation of N-terminal peptide antibody

(A) Synthesis of a peptide having 20 residues at the N-terminus

A peptide was synthesized shown in Figure 18 (a) and having the amino acid sequence of the N-terminal 20 residues peptide of human osteocalcin and having cystein as the 21st residue.

The synthesis was carried out using a peptide synthesizer produced by ABI Co. The peptide was named Ost-N(20).

(B) Preparation of an antigen (a bound product of Ost-N(20) and carrier protein)

Keyhole limplet hemocyanin (KLH) was used as a carrier protein, and KLH was MBS acylated with N-(m-maleimidobenzoic acid)-N-succinimide ester (MBS). On the other hand, Ost-N(20) was treated with 2-mercaptoethanol to make the SH group free, and reaction was carried out by dropwise adding the Ost-N(20) to the MBS acylated KLH while the reaction mixture was maintained at pH 6.0 to 6.5. After the reaction for 3 hours, the reaction mixture was dialyzed, and the obtained product was used as an antigen.

(C) Preparation of antibody

A rabbit was immunized with 500 µg/l shot of the KLH bound product of Ost-N(20).

Since the antibody titer increased after the immunization was repeated 6 times at an interval of 2 weeks, blood was withdrawn therefrom, and the antibodies were purified by protein A-Sepharose to obtain an objective antibody.

Reference example 2 Preparation of C-terminal peptide antibody - (1)

(A) Synthesis of a peptide having 7 residues at the C-terminus

A peptide shown in Figure 18 (b) and having the amino acid sequence of the C-terminal 7 residues peptide of human osteocalcin was synthesized using a peptide synthesizer produced by ABI Co.

The peptide was named Ost-C(7).

(B) Preparation of an antigen (a bound product of Ost-C(7) and a carrier protein)

Ost-C(7) and KLH were admixed in the same weight with each other, and dicyclo-hexylcarbodiimide (DCC) was reacted therewith to prepare a bound product of Ost-C(7) and KLH.

The obtained product was purified by gel filtration of HPLC.

(C) Preparation of an antibody

A rabbit was immunized with 500 µg/body shot of the KLH bound product of Ost-C(7). Thereafter, the same operation as in Reference example 1 was made to obtain an objective antibody.

Reference example 3 Preparation of C-terminal peptide antibody - (2)

(A) Peptide synthesis of C-terminal 15 residues

A peptide was synthesized shown in Figure 18 (C) and having the amino acid sequence of the C-terminal 15 residues peptide of human osteocalcin.

The synthesis was carried out using a peptide synthesizer produced by ABI Co. The peptide was named Ost-C(15).

(B) Bonding of Ost-C(15) to a carrier protein

A bound product of Ost-C(15) and KLH was prepared using the same method as in (B) of Reference example 2.

(C) Preparation of an antibody

An objective antibody was obtained by the same method as in (C) of Reference example 2 using the bound product of Ost-C(15) and KLH as an antigen.

Reference example 4 composition of the assay system

(A) Preparation of horseradish peroxidase (HRP)-labeled antibody

(1) HRP labeling of the anti-Ost-N(20) antibody (IgG)

50 µl of a dimethylformamide solution of 10 mg/ml MBS was added to 2 ml of a 0.01M phosphoric acid and 0.15M NaCl solution (pH 7.4) of 1 mg/ml the anti-Ost-N(20) antibody, and reaction was carried out at 25°C for 30 minutes. Then, gel filtration was carried out using a column packed with Sephadex G-25 and using 0.1M phosphate buffer (0.1M PB) (pH 6.0) to separate the MBS acylated antibody from the unreacted MBS.

In the other hand, 120 µl of a dimethylformamide solution of 60 mg/ml S-acetylmercaptosuccinic anhydride was added to 2 ml of a 0.1M PB solution (pH 6.5) of 10 mg/ml HRP, and reaction was carried out at 25°C for 2 hours. Then were added 800 µl of a 0.1M Tris-HCl buffer (pH 7.0), 160 µl of 0.1M EDTA and 1.6 ml of 1M hydroxylamine, followed by reaction at 0°C for 4 minutes. Thereafter, the reaction solution was placed in a collodion bag and dialyzed at 4°C for 3 days against 0.1M PB (pH 6.0) containing 5 mM EDTA to obtain mercaptosucciny HRP.

Then, 2 mg of the MBS acylated antibody and 4 mg of the mercaptosuccinyl HRP were mixed, concentrated with ice cooling using a collodion bag until the protein concentration of 4 to 10 mg/ml was obtained, and allowed to stand overnight at 15 to 20°C. The solution was subjected to gel filtration using a column packed with Ultrogel AcA 44 (Pharmacia LKB Co.) to obtain an HRP-labeled anti-Ost-N(20) antibody.

(2) HRP labeling of anti-Ost-N(20) antibody (F(ab')$_2$)

To 1 ml of a PBS solution of 2.0 mg/ml the anti Ost-N(20) antibody were added 100 µl of a 1M acetate buffer (pH 4.2) and a solution obtained by dissolving 40 µg of pepsin in 20 µl of the buffer, followed by reaction at 37°C for 4 hours. After the complection of the reaction, F(ab')$_2$ was recovered by separation using a Sephadex G25 column (Ø 2 cm x 45 cm) equilibrated with PBS. An HRP-labeled anti-Ost-N(20) (F(ab')$_2$) antibody was prepared in the same manner as in Reference Example 1(A)(1).

(3) HRP labeling of the anti-Ost-N(20) antibody F(ab')$_2$

The F(ab')$_2$ fraction of the anti-Ost-N(20) antibody was prepared in the same manner as in Reference Example 1(A)(2), and reduced with mercaptoethylamine, followed by gel filtration HPLC using a G3000SW column produced by Toso Co., Ltd. to purity the Fab'.

On the other hand, HRP was MBS acylated in the same manner as in the MBS acylation of the anti-Ost-N(20) antibody in Reference Example 1(A)(1). Then 2 mg of the anti-Ost-N(20) antibody (Fab') and 4 mg of the MBS acylated HRP were admixed, concentrated using FILTRON (an ultrafiltration apparatus) and subjected to reaction at 4°C for 16 hours. The reaction solution was subjected to isolation and separation by gel filtration HPLC using a Toso G3000SW column to obtain an HRP-labeled anti-Ost-N(20)(Fab') antibody.

(B) Preparation of beads immobilizing an antibody

(1) Immobilization of an antibody

Polystyrene beads (diameter 6 mm) after sufficient washing were left standing at a temperature of 4°C overnight in a PBS solution containing the anti-Ost-C(7) or anti-Ost-C(15) in a concentration of 20 µg/ml, washed with PBS, and then subjected to post-coating treatment by leaving them standing at a temperature of 4°C overnight in a PBS solution of 1 % bovine serum albumin (BSA) to obtain beads immobilizing the anti-Ost-C(7) antibody or anti-Ost-C(15) antibody.

(2) Activity evaluation of the beads immobilizing the antibody

The activity of the anti-Ost-C(7) antibody and anti-Ost-C(15) antibody prepared in the above (1) was investigated by the following method. To test tubes were added respectively each one of the beads immobilizing each antibody, 200 µl portions of 0.15M Tris-HCl-0.15M NaCl buffers (0.05M TBS) (pH 8.0) containing 1 % BSA and purified human osteocalcin (a standard substance) in the range of 0 to 100 ng/ml, and 200 µl portions of a 0.05 M TBS (pH 8.0) solution containing 1 % BSA and the Fab' fraction of the HRP-labeled anti-Ost-N(20) antibody, followed by incubation at 4°C for 17 hours. The solution in each test tube was then removed by aspiration, washing was conducted with 0.05M TBS (pH 8.0), to each test tube was added 0.4 ml of a 0.1M phosphate/citrate buffer (pH 4.3) containing 0.02 % 3,3',5,5',tetramethylbenzidine and 2.5 mM $H_2O_2$, reaction was carried out at a temperature of 25°C for 30 minutes, and the enzymatic reaction was stopped by the addition of 1 ml of a 1N aqueous sulfuric acid solution as a reaction-discontinuing agent.

Then, the absorbance of the resulting solutions were measured respectively at a wavelength of 450 nm using a spectrophotometer and plotted in correspondence to the standard substance concentrations of 0 to 100 ng/ml. The results are shown in Table 19.

(C) Composition of the assay system

To test tubes were added each one of the beads immobilizing the anti-Ost-C(7) antibody and prepared in Reference Example 1(B)(1), 200 μl portions of 0.05M TBS (pH 8.0) buffers containing 1 % BSA and purified human osteocalcin (a standard substance) in the range of 0 to 20 ng/ml, and 200 μl portions of 0.05M TBS (pH 8.0) solutions containing 1 % BSA and respectively the various HRP-labeled antibody prepared in Reference example 4(A)(1) to (3), in each combination of the bead immobilizing the antibody with the IgG and $F(ab')_2$ fractions of the HRP-labeled antibody, and then incubation was carried out at 25°C for 2 hours. The solution in each test tube was removed by aspiration, washing was conducted with 0.05 M TBS (pH 8.0), to each test tube was added 0.4 ml of a 0.1M phosphate/citrate buffer (pH 4.3) containing 0.02 % 3,3',5,5'-tetramethylbenzidine hydro chloride and 2.5 mM $H_2O_2$, reaction was carried out at a temperature of 25°C for 30 minutes, and then 1 ml of a 1N aqueous sulfuric acid solution was added as a reaction-discontinuing agent to stop the enzymatic reaction. The absorbance of the resulting solutions were measured at a wavelength of 450 nm using a spectrophotometer and plotted in correspondence to the standard substance concentrations of 0 to 20 ng/ml, and thereby were calculated N/S ratios on the fractions of the HRP standard antibody (values obtained by dividing the absorbance at an antigen concentration of 0 ng/ml by the absorbance at an antigen concentration of 20 ng/ml). The results are shown in Table 1.

Table 1

Assay of human osteocalcin

| Subtype of HRP-labeled antibody | $OD_{450}$ (osteocalcin) 0 ng/ml) | $OD_{450}$ (osteocalcin 20 ng/ml) | N/S ratio (%) |
|---|---|---|---|
| IgG | 0.050 | 0.700 | 7.1 |
| F(ab')2 | 0.040 | 0.630 | 6.3 |
| Fab' | 0.070 | 0.640 | 10.9 |

It is apparent from Table 1 that assay sensitivity varies depending on the subtype of the HRP-labeled antibody and the $F(ab')_2$ fraction and IgG fraction have a low background and constitute a more highly sensitive assay system.

Figure 20 shows the calibration curve when the HRP-labeled antibody ($F(ab')_2$) was used.

Assay was carried out with the thus composed intact human osteocalcin assay system (the HRP-labeled antibody is $F(ab')_2$) on the medium fraction, the periplasmic fraction, the cytoplasmic fraction and the outer membrane-inner membrane-inclusion body fraction 10 to 20 times diluted respectively (as the diluting liquid was used 0.1 % BSA-0.2 % skim milk-0.02 % Tween 20-0.05M TBS (pH 7.4)).

Example 1

Design, synthesis and subcloning of a human osteocalcin-expressing DNA sequence

The design of a human osteocalcin-expressing DNA sequence was made by using as a base the amino acid sequence of human osteocalcin shown in Figure (A. J. Celeste et al., J., 5, 1885-1890 (1986)) and providing restriction enzyme recognition sites necessary for gene construction. The design is shown in Figure 6. In the figure, the solid lines denote the boundaries of the synthesized DNA fragments and the dotted lines denote restriction enzyme cleavage.

The human osteocalcin-expressing DNA sequence (shown in Figure 7) roughly consists of 4 parts, namely the part encoding the signal peptide (prepeptide) (sequence A in the figure), the propeptide part [the part encoding the part recognized by an enzyme capable of Glu to Gla conversion on human osteocalcin (sequence B in the figure) + the part encoding the cleavage sequence part (sequence C (in the figure)] and the part encoding the human osteocalcin (sequence D in the figure). The signal peptide part, the part recognized by the enzyme of capable of Glu to Gla conversion, the cleavage sequence part and the human osteocalcin part can be replaced in cassette style by the respective parts having another sequence by cleavage with proper restriction enzymes.

The thus designed gene was divided at the boundary positions of the synthetic DNA fragments shown in Figure 6, and in total 12 oligonucleotides ( ① to ⑫ ) were chemically synthesized.

The synthesis of the oligonucleotides was carried out by the phosphoramidite method using a full automatic DNA synthesizer (Applied Biosystems, Inc., model 381A). The purification of the synthesized oligonucleotides was carried out according to the manual of Applied Biosystems, Inc. Namely, each of the aqueous ammonia solutions of the synthesized oligonucleotides was held at 55° C overnight to remove the protective group(s) of the DNA base, and a synthesized oligonucleotide fraction having a high molecular weight was obtained by gel filtration using Sephadex G-50 fine gel (produced by pharmacia Co.).

Then, after polyacrylamide gel electrophoresis containing 8M urea (gel concentration 10 %), electrophoresis pattern was observed by the ultraviolet ray shadowing method, the band corresponding to the objective oligonucleotide part was cut, the polyacrylamide gel fragment was finely fractured, to the fractured fragment was added 5 ml of a buffer for elution (500 mM $NH_4OAc$-1 mM EDTA-0.1 % SDS (pH 7.5)), and the mixture was shaked at 37° C overnight. Then, the aqueous layer containing the objective DNA was recovered by centrifugation. Finally, the solution containing the synthesized oligonucleotide was passed through a gel filtration column (Sephadex G-50) to obtain the purified synthesized oligonucleotide. If necessary, the purify of the synthesized oligonucleotide was enhanced by repeating the polyacrylamide gel electrophoresis. The thus obtained purified synthesized oligonucleotides (1 to 10 μg) were subjected respectively to polynucleotide kinase reaction in the presence of 1 mM ATP to phosphorylate the 5' terminal side.

The polynucleotide kinase reaction was carried out in an aqueous solution of 50 mM Tris-HCl (pH 9.5)-10 mM $MgCl_2$-5 nN dithiothreitol using 10 units of T4 polynucleotide kinase. Then, two of the synthesized oligonucleotides were admixed whose 5'-terminus was phosphorylated and which correspond to the upper chain and the lower chain respectively, and annealing was carried out by gradually cooling the aqueous solution from a temperature of 95° C to room temperature.

Ligation reaction was carried out with T4DNA ligase using 3 μg each of the the synthesized fragments after the annealing ( ⑦ , ⑧ ), ( ⑨ , ⑩ ) and ( ⑪ , ⑫ ) (These 6 synthesized fragments constitute sequence A-B-C among the above sequence A-B-C-D (Figure 6)). After this reaction solution was subjected to phenol/chloroform treatment and ethanol precipitation, separation was made by 2 % agarose electrophoresis, and the band of about 150 bp was recovered from the gel according to Method 4.

For subcloning was used a modified body pBPR9, a modified plasmid of a cloning vector pHSG399 (Takara Shuzo Co., Ltd.). This preparation was made as follows.

First pHSG399 was cleaved with a restriction enzyme PvuI, blunt ended by repair reaction according to Method 6 and then subjected to intramolecular ligation reaction with T4DNA ligase according to Method 5 to prepare pHSG-PR9 having no PvuI site.

Then, pHSG-PR9 was cleaved with BglI, blunt ended by the repair reaction and then subjected to the intramolecular ligation reaction with T4DNA ligase to prepare pBPR9 having no BglI site (Figure 8).

(both pHSG-PR9 and pBPR9 produce a peptide holding the activity of the α chain of β-galactosidase, and the XLI-Blue transformant forms a blue colony on the IPIG-Xgal agar plate).

The thus obtained pBPR9 was cleaved with EcoRI and KpnI, the cleaved fragments were separated by 1 % agarose electrophoresis (Method 2), and the objective DNA fragment was recovered from the gel according to Method 3. The recovered DNA fragment and the 150 bpDNA fragment corresponding to the sequence A-B-C were ligated with T4DNA ligase according to Method 5.

The resulting ligation reaction solution was added to 200 μl of Escherichia coli XLI-Blue competent cells prepared by Method 7 to perform transformation, and the resulting cells were inoculated on the selection agar plate containing chloramphenicol pBPR-PRE was prepared as a plasmid DNA from the colonies grown on the selection agar plate (Figure 9).

The formation of the plasmid pBPR-PRE having the objective subclone was ascertained by agarose gel electrophoresis that the plasmid was cleaved with AvaIII and when it was cleaved with EcoRI and PstI, a fragment of about 150 bp was observed.

By using the same procedures as above were carried out the annealing, ligation reaction and DNA fragment recovery of the synthesized fragments ①, ②, ③, ④, ⑤ and ⑥ corresponding to sequence D part.

On the other hand, the cloning vector pBPR9 was cleaved with KpnI and XbaI, the DNA fragments were separated by agarose electrophoresis and the objective DNA fragment was recovered from the gel according to Method 3.

The recovered DNA fragment and the DNA fragment (about 150 bp) corresponding to sequence D part were ligated according to Method 5, and then transformation was made according to Method 7 to obtain chloramphenicol resistant colonies. These resistant colonies were liquid cultured, and the plasmids were respectively prepared therefrom, cleaved with KpnI and XbaI and then subjected to agarose electrophoresis, and the band of about 150 bp was observed, whereby a plasmid pBPR-MOC having the objective DNA fragment (sequence D) was obtained (Figure 10).

Example 2

Cloning of a human osteocalcin-expressing DNA sequence and ascertainment of its DNA sequence

The plasmid pBPR-PRE cloning the sequence A-B-C part of the human osteocalcin-expressing DNA sequence obtained in Example 1 was cleaved with restriction enzymes EcoRI and KpnI and then subjected to separation by agarose electrophoresis, and the DNA fragment of about 150 bp corresponding to sequence A-B-C part was recovered from the gel by Method 4.

On the other hand, the plasmid pBPR-MOC obtained in Example 1 and cloning the sequence D part of the human osteocalcin-expressing DNA sequence was cleaved with restriction enzymes EcoRI and KpnI, the cleaved DNA fragments were separated by agarose electrophoresis, and the DNA fragment of about 2350 bp containing sequence D and the vector was recovered from the gel according to Method 3.

Ligation of the former about 150 bp DNA fragment and the latter about 2350 bp DNA fragment was carried out according to Method 5 and then, transformation was carried out according to Method 7, and plasmids were prepared from the chloramphenicol resistant colonies. Each of the plasmid DNAs obtained from the several colonies was cleaved with restriction enzymes EcoRI and PstI, the cleaved fragments were separated by agarose electrophoresis, and by accertaining the fragment of about 300 bp was obtained a plasmid pBPR-OC having the objective human osteocalcin-expressing DNA sequence (sequence A-B-C-D) (Figure 11).

Example 3

Preparation of expression plasmid

The plasmid pBPR-OC obtained in Example 2 was cleaved with a restriction enzyme PvuI, subjected to ethanol precipitation and then subjected to blunt ending reaction according to Method 6. The resulting cleaved and blunt ended plasmid was cleaved with a restriction enzyme PstI, and the DNA fragment of about 300 bp (the human osteocalcin-expressing DNA sequence part) was separated by agarose electrophoresis and recovered by Method 4.

On the other hand, the expression vector pKK233-2 (produced by Pharmacia Co.) was cleaved with NcoI and subjected to ethanol preciptation, and the precipitate was subjected to blunt ending reaction according to Method 6. The resulting substance was cleaved with PstI and subjected to agarose electrophoresis to separate a DNA fragment of about 4600 pb, which was then recovered by Method 3.

The thus obtained former DNA fragment of about 300 bp and the latter DNA fragment of about 4600 pb were ligated according to Method 5. Transformation was carried out according to Method 7 using the resulting ligation reaction solution, and plasmids were prepared from the colonies grown on the selection agar medium containing ampicillin (50 $\mu$g/ml). The obtained plasmid DNAs were cleaved with restriction enzymes EcoRI and Pst I, and by ascertaining the DNA fragment of about 600 bp by agarose electrophoresis an expression plasmid pKK-OC was obtained (Figure 12).

Example 4

Ascertainment of the production of a human osteocalcin-containing polypeptide by colony hybridization

Host cells XLI-Blue were transformed with the expression plasmid pKK-OC obtained in Example 3. The resulting transformant (recombinant host cells) XLI-Blue [pKK-OC] was inoculated in the LB medium and cultured at 37°C overnight. The culture broth (about $10^9$ cells/ml) were $10^{-6}$ diluted, and 0.05 ml of the diluent was inoculated on a nitrocellulose membrane mounted on the LB agar plate (ampicillin 50 $\mu$g/ml). On the next day, when the diameters of the generated colonies became about 1 mm, the membrane was peeled off (colonies were formed on the membrane), 2 ml of the LB medium (containing 2 mM PITG) was spread on the agar plate, and the membrane was mounted again thereon to give induction. 8 hours, later, the membrane was peeled off, and exposed to chloroform vapor to lyze the cells and at the same time make the bacterial proteins adhere on the membrane. Thereafter, blocking was carried out at 4 °C overnight using 3 % BSA (bovine serum albumin)-20 mM PBS. On the next day, each membrane was washed twice with 0.05 % Tween 20-20 mM PBS at room temperature for 2 hours. The resulting membrane was immersed in 1 % BSA-20 mM PBS containing in a concentration of 1 μg/ml the HRP-labeled antibody (one obtained in Reference example 4) of the rabbit polyclonal antibody (one obtained in Reference example 1) against a synthesized peptide homologous to the N-terminal 20 residues peptide of human osteocalcin, and incubated at 4 °C overnight. Thereafter, the membrane was washed twice again with 0.05 % Tween 20-20 mM PBS at room temperature for 20 minutes, and then washed with 20 mM PBS at room temperature for 20 minutes. Finally, coloring reaction was performed for 4 minutes using Konica Immunostain (produced by KONICA CORPORATION). As a result, as for the transformant XLI-Blue [pKK-OC] harboring the expression plasmid pKK-OC the colony part was stained dark blue, whereas the transformant XLI-Blue [pKK233-2] harboring the expression vector pKK233-2 was not stained. It was ascertained by this that the transformant XLI-Blue [pKK-OC] produced a polypeptide containing human osteocalcin.

Example 5

Determination of the production site and production amount of the human osteocalcin-containing polypeptide by enzyme immunoassay (EIA)

The transformant harboring the expression plasmid pKK-OC obtained in Example 3 was inoculated in the LB medium (ampicillin 50 μg/ml) and cultured at 37 °C overnight. 0.4 ml of the culture broth was inoculated in 40 ml of 2XYT medium (1.6 % trypton-1.0 % yeast extract-0.5 % NaCl (pH 7.6)) and cultured at 37 °C. When the absorbance at a wavelength of 600 nm ($OD_{600}$) became about 0.8 (the logarithmic phase), 400 μl of a 100 mM aqueous IPTG solution (final concentration 1 mM) was added to give induction. Part of the culture broth was taken immediately before the induction and 1, 3 and 5 hours after the induction respectively, and they were preserved at 0 °C.

The used transformants are two kinds, i.e. XLI-Blue [pKK-OC] and XLI-Blue $F^-$[pKK-OC] (This is a F factor deficient strain of XLI-Blue and does not have repressor lac I), and as for XLI-Blue [pKK-OC] were used both one subjected to induction with IPTG (isopropyl-β-D-thio-galactopyranoside) and one not subjected thereto.

As for the taken samples, in order to make each cell number equal, [2.4 ÷ ($OD_{600}$ value)] ml portions of the culture brothes were taken, and the cells were respectively collected (centrifugation 5000 rpm, 4 °C, 5 minutes) and the supernatants (the medium fractions) were preserved at -20 °C. Each pellet (cells) was separated according to Method 10 into the periplasmic fraction, the cytoplasmic fraction, the outer membrane-inner membrane-inclusion body fraction.

The amount of the human osteocalcin-containing polypeptide (hereinafter, the human osteocalcin-containing polypeptide is represented by "hOC▲ ") in the thus obtained samples was assayed by EIA (Method 11). The results are shown in Table 2.

Table 2

| Transformant | t | HOC▲ per 2.4 $OD_{600}$·ml (ng) | | | |
|---|---|---|---|---|---|
| | | S | P | C | M |
| XL1-Blue [pKK-OC] IPTG-induction | 0 | <0 | 3.30 | 2.34 | 0.80 |
| | 1 | <0 | 4.02 | 2.70 | 1.20 |
| | 3 | 0.0 | 1.92 | 3.27 | 1.62 |
| | 5 | 0.09 | 1.38 | 2.64 | 1.61 |
| XL1-Blue $F^-$ [pKK-OC] | 0 | <0 | 7.73 | 4.17 | |
| | 1 | <0 | 3.95 | 3.23 | |
| | 3 | 0.15 | 4.25 | 3.90 | |
| | 5 | <0 | 1.76 | 2.93 | |
| XL1-Blue [pKK-OC] | 0 | <0 | 4.10 | 2.60 | |
| | 1 | <0 | 2.33 | 2.90 | |
| | 3 | <0 | 1.08 | 1.92 | |
| | 5 | <0 | 0.68 | 2.07 | |

t: Lapse time is shown after the induction or after the cell turbidity became $OD_{600} \mp 0.8$

S: Medium fraction

P: Periplasmic fraction

C: cytoplasmic fraction

M: the outer membrane.inner membrane. inclusion body fraction

These results show that, at the logarithmic phase of the transformant XLI-Blue [pKK-OC], hOC▲ exists in the distribution of 0 % in the medium fraction, 50 % in the periplasmic fraction, 35 % in the cytoplasmic fraction and 15 % in the outer membrane.inner membrane. inclusion body fraction, and in the largest amount in the peripCasmic fraction (P). From this fact it is suggested hOC▲ was secreted in a form such that the signal peptide (the part encoded by sequence A) was cut off, i.e. in the form of B'-C'-D' (the general formula [II]).

Further, it was found that hOC▲ was produced in the largest amount at the logarithmic phase in the transformant XLI-Blue $F^-$ [pKK-OC] of XLI-Blu $F^-$, a strain not containing lac I (repressor protein).

Example 6

Preparation of a modified signal peptide-expressing plasmid

A modified signal peptide was prepared wherein the charge of the N-terminus of the signal peptide of human osteocalcin was changed to +2.

As shown in Figure 13, the synthesized DNA fragments ⑬ and ⑭ were prepared in the same manner as in Example 1, and phosphorylation and annealing were then performed. On the other hand, the plasmid pBPR-OC cloning the human osteocalcin-expressing sequence was cleaved with restriction enzymes EcoRI and BglI and separated by agarose electrophoresis, and the DNA fragment about 2500 bp was recovered by Method 3. Thus obtained synthesized DNA fragment ⑬ + ⑭ and DNA fragment of about 2500 bp were ligated according to Method 5. Transformation was carried out with the resulting ligated DNA fragment according to Method 7, the plasmid DNAs obtained from the chloramphenicol resistant colonies were cleaved respectively with DraI, and by observing the agarose gel electrophoresis patterns of the resulting DNA fragment(s), a plasmid pOC 28 (Figure 14) was obtained having the DNA sequence of the modified signal peptide.

The obtained plasmid pOC28 was cleaved with restriction enzymes DraI and PstI, and the obtained about 300 bp DNA fragment (containing the human osteocalcin-expressing DNA sequence) was recovered by Method 4. On the other hand, in the same manner as in Example 3, the expression plasmid vector pKK233-2 was cleaved with a restriction enzyme NcoI, subjected to repair reaction, cleaved with a restriction enzyme PstI, and an about 4600 bp DNA fragment was recovered. The above ca. 300 bp DNA fragments and the ca. 4600 bp DNA fragment were ligated according to Method 5, and then according to Method 7, transformation was carried out and plasmid DNAs were isolated from the ampicillin resistant colonies. The plasmid DNAs obtained from the several resistant colonies were cleaved respectively with a restriction enyme BamHI, a DNA fragment about 430 bp was ascertained by agarose electrophoresis, and a human osteocalcin-expressing plasmid pKOC28 was obtained (Figure 15). The hOC$^{▲}$ produced by this plasmid has a lysine residue at the N-terminus of the signal peptide and the charge becomes +2 in total with the arginine residue.

Example 7

Comparison in the production amount in transformants of the human osteocalcin-expressing plasmid pKK-OC with its modified plasmid pKOC28

According to Example 5, as for the following transformants, the production amount of hOC$^{▲}$ in the periplasmic fraction was assayed by intact human osteocalcin EIA.

Used transformants HBl0l [pKK-OC]
HBl0l [pKOC28]
XLl-BlueF$^{-}$ [pKOC28]
PAM660 [pKOC28]
The results are shown in Tables 3 and 4.

It was found from these results that the transformant XLl-BlueF$^{-}$ [pKOC28] produces the largest amount of hOC$^{▲}$ at the logarismic phase.

This Escherichia coli K-12 strain XLl-BlueF$^{-}$ [pKOC28] containing no lac I was deposited with Fermentation Research Institute, Agency of Industrial Science and Technology under the accession number of Ferm BP-2950 on June 13th, 1990.

Table 3

Comparison in the production amount in transformants from the host HB101 strain of the human osteocalcin-expressing plasmid pKK-OC with its modified plasmid pKOC28

| | hOC▲ (ng) in the periplasmic fraction per $10^9$ cells | |
|---|---|---|
| | logarismic phase | stationary phase |
| HB101 [pKK-OC] | 0.33 | 0.13 |
| HB101 [pKOC28] | 2.88 | 0.55 |

Table 4

Comparison in the hOC▲ production amount in different hosts of the modified plasmid pKOC28 of the human osteocalcin-expressing plasmid

| | hOC▲ (ng) in the periplasmic fraction per $10^9$ cells | |
|---|---|---|
| | logarismic phase | stationary phase |
| XL1-BlueF$^-$ [pKOC28] | 7.7 | 4.3 |
| PAM660[pKOC28] | 5.7 | 5.7 |
| HB101 [pKOC28] | 2.6 | 0.9 |

Example 8

Investigation of immune reactivity

The transformant XLI-BlueF$^-$ [pKOC28] was cultured, and the periplasmic fraction was prepared by Method 10.

2 units of bovine thrombin (produced by Mochida Pharmaceutical Co., Ltd.) was added to 1 ml of the periplasmic fraction, and incubation was carried out at 37°C for 1 hour. A dilution test was carried out on the thus obtained thrombin-digested sample and a thrombin-untreated periplasmic fraction. This method was carried out, likewise in Examples 5 and 7, using the intact human osteocalcin EIA (Method 11).

The results are shown in Figure 16. The dilution curve (the straight line b in the figure) of the periplasmic fraction containing hOC▲ (B'-C'-D') produced by the transformant is on a parallel with the standard substance, i.e. human osteocalcin wherein Glu to Gla conversion is made (the straight line a in the figure), which suggests that this hOC▲ is usable as a standard substance. Further, about the thrombin-digested sample wherein it is surmised that sequence B'-C' was cleaved (the sample contains sequence D', namely human osteocalcin wherein Glu to Gla conversion is not made; the straight line c in the figure), the value by EIA is heightened compared to hOC▲ (B'-C'-D'). This fact suggests that sequence B'-C' sterically hinders the reaction of the antibody with the N-terminus of human osteocalcin.

Example 9

Purification of a human osteocalcin-containing polypeptide by a immuno affinity column

The transformant XLI-BlueF⁻ [pKK-OC] was cultured in 500 ml of the LB medium at 37°C overnight. The culture was inoculated in 10 l of the M9CA modified medium (2 % Casamino acid, 0.2 % yeast extract, 0.1 % $NH_4Cl$, 0.02 % $MgCl_2$, 0.01 % $CaCl_2$, 0.0017 % vitamin B1, 0.25 % glucose) in a jar-fermentor, and cultured at 37°C. 8 hours later, culturing was stopped at the time when the turbidity of the culture broth became $OD_{660} \mp 6$, and the cells and the medium were separated by continuous centrifugation (15,000 rpm, 15°C, 1 hour).

When the separated supernatant was assayed by EIA according to Method 11, the hOC▲ concentration of 5 to 7 ng/ml was obtained. 1 l of the supernatant was passed at a flow rate of 0.2 ml/min through a Sepharose 4B column immobilizing a polyclonal antibody (PCA) against a synthesized peptide of the human osteocalcin N-terminal 20 residues, and the column was washed with about 20 ml of PBS until the absorbance at 280 nm of the effluent became 0.

Elution was then carried out with 6 ml of 0.1M glycine-HCl (pH 2.5) (flow rate: 1 ml/8 to 9 minutes), and the eluate was taken in 1 ml portions. 1 ml portions of 0.2M Tris-HCl (pH 8.5) were placed in advance in the test tubes for the eluate.

The obtained samples were assayed for the concentration of hOC▲ by intact human osteocalcin EIA according to Method 11. The results are shown Figure 17.

It is seen from Figure 17 that human osteocalcin and the human osteocalcin-containing peptide (hOC▲ ) could be purified by the antibody affinity column. After the purification, a human osteocalcin wherein Glu to Gla conversion is made and which has physiological activities can be obtained by performing Glu to Gla conversion in vitro, for example with the liver-derived γ-carboxylase.

Example 10

Design of a human growth hormone (hGH)-human osteocalcin (hOC) fused protein-expressing DNA sequence and synthesis of a Linker DNA

As shown in Figure 21 was designed the construction of a human growth hormore-human osteocalcin fused protein-expressing plasmid. Further the Linker DNA part was designed as shown in Figure 22. The 5'-terminus can be ligated to the BamH I cleavage site and the Bgl II cleavage site. The 3'-terminus can be ligated to the Bbe I cleavage site. Further, the amino acid sequence Ile-Glu-Gly-Arg is a cleavage sequence with FXa and Lys-Pro-Ser-Gly corresponds to the N-terminal 4 residues of sequence B. The ligation DNA was synthesized using a process similar to that of example 1 about the case of the + strand and the - strand, respectively. The synthesized DNA was purified using an oligonucleotide-purifying cartridge (produced by Applied Biosystems Japan Inc.) and the operation procedure described in the manual therefor. 5 μg of the + strand and 5 μg of the - strand were admixed, heated at 70°C for 5 minutes in an annealing solution (0.1M NaCl-10 mM Tris.HCl (pH 8.0-1mM EDTA), incubated at 50°C for 2 hours and left overnight to bring the temperature back to room temperature, whereby annealing was carried out. The resulting solution was evaporated to dryness and the resulting DNA was preserved.

Ascertainment of the human osteocalcin-containing polypeptide hOC▲ by enzyme immunoassay

The periplasmic fraction was prepared from XLI-BlueF⁻ [pKOC28] by the method C.

In order to ascertain that hOC▲ has the structure of B'-C'-D' having sequence B', a rabbit was immunized with the human osteocalcin propeptide (Figure 5) using an usual known method to obtain a polyclonal antibody against the human osteocalcin propeptide. Enzyme immunoassay was conducted by a sandwich system using the polyclonal antibody. The sandwich system with the beads immobilizing anti-Ost-C(7) antibody was conducted by a sandwich system using the polyclonal antibody. The sandwich system was composed by the beads immobilizing anti-human osteocalcin propeptide polyclonal antibody and HRP-labeled anti-Ost-C(7) (Fab') polyclonal antibody (Reference Example 2) according to the method of Reference Example 4. As a result of the assay, a dilution curve similar to curve (b) in Figure 16 was obtained and thereby it was ascertained that hOC▲ really has sequence B'.

Example 11

Preparation of a fused expression plasmid of the ($Met^1$-$Phe^{130}$) human growth hormone fragment polypeptide and the human osteocalcin (B-C-D) sequence part

1 μg of the human growth hormone expression plasmid pGH-L9 (M Ikehara et al., P.N.A.S., 81, 5956 (1984)) was cleaved with restriction enzymes Bgl II and Sal I, the resulting fragments were separated by agarose gel electrophoresis (Method 2), and the DNA fragment of about 4.1 kb was recovered from the gel (Method 3).

Further, the plasmid pBPR-OC obtained in Examples 1 and 2 was cleaved with restriction enzymes Bbe I and Sal I, the fragments were separated by agarose gel electrophoresis (Method 2), and the DNA fragment of about 220bp was recovered from the gel (Method 4).

The above recovered two DNA fragments and the DNA fragment synthesized in Example 10 were ligated according to Method 5. Further, transformation was carried out with the reaction solution according to Method 7 to obtain ampicillin resistant colonies. These resistant colonies were liquid cultured, the plasmids were prepared from the resulting cultures, respectively, cleaved with a restriction enzyme Kpn I and then subjected to agarose gel electrophoresis, and the plasmid on which it was ascertained that it was cleaved with Kpn I was named a fused expression plasmid pGH-POCI (Figure 23).

Further, the same procedure as above was conducted except that a DNA fragment obtained by cleaving pGH-L9 with restriction enzymes BamH 1 and Sal I was used in place of the above DNA fragment obtained by cleavage with Bgl II and Sal I, whereby a fused expression plasmid pGH-POCII was obtained (Figure 24).

Example 12

Expression of the fused protein gene

Host cells HBl0l and JMl09 were transformed with the fused expression plasmids pGH-POCI and pGH-POC II obtained in Example 11. The resulting 4 transformants HBl0l [pGH-POCI], HBl0l [pGH-POCII], JMl09 [pGH-POCI] and JMl09 [pGH-POCII] were cultured as follows and then was conducted acrylamide gel electrophoresis (SDS-PAGE) (U.K. Leammli, Nature, 227, 680 (1970)).

The transformants were inoculated respectively in portions of the LB medium containing 50 to 100 μg/ml ampicillin and cultured at 37°C overnight. The overnight cultures were added in an amount of 1/100 respectively to portions of the M9 medium (a medium prepared by autoclave sterilizing an aqueous solution of 0.6 % $Na_2HPO_4$-0.3 % $KH_2PO_4$ -0.05 % NaCl-0.1 % $NH_4Cl$ (pH 7.4) and adding thereto an aqueous $MgSO_4$ solution and an aqueous $CaCl_2$ solution separately autoclave sterilized so that the respective final concentrations become 2 mM and 1 mM), and cultured at 37°C. When the turbidity of each transformant became $OD_{600} \fallingdotseq 0.5$, 3-β-indoleacrylic acid (IAA) was added to each culture broth so as to make its final concentration 40 μg/ml, and culturing was further continued until the proliferation of the cells was stopped. Several milliliters of each culture broth was taken 0 and 2 hours after the addition of IAA and thereafter every 1 hour, and preserved in a freezed state. The results of SDS-PAGE carried out on them are shown in Figure 25. (A) in the figure shows the results when the plasmid pGH-POC I was used and (B) shows the results when the plasmid pGH-POCII was used. It is seen that the fused proteins were formed respectively at the part of the arrow (MW was about 28,000 in Figure (4) and MW about 26,000 in Figure (B)). The production amounts were respectively on the order of several percent of the total protein amounts of the cells, the transformants carrying the fused expression plasmid pGH-POCI produced a larger amount of the fused protein, and the transformants of the HBl0l strain had a higher proliferation rate than that of the transformants of the JMl09 strain.

The transformant HBl0l [pGH-POCI] was deposited with Fermentation Research Institute, Agency of Industrial Science and Technology on November 27th, 1990 under accession number of FERM BP-3175.

Example 13

Purification of the fused protein

The transformant HBl0l [pGH-POCl] was cultured in the same manner as in Example 12; the cells were collected by centrifugation, suspended in 0.5 M Tris-MCl (pH 8.0) and fractured using an ultrasonic fracturing apparatus (produced by Kubota Shoji Co., Ltd., 200 M type); and the mixture was centrifuged to obtain the objective fused protein as an inclusion body in the precipitated part. The precipitate was resuspended in 0.5 M Tris-HCl (pH 8.0), subjected to ultrasonic fracture and then centrifuged to remove from the precipitate an as large as possible amount of the membrane protein components. The amount of the obtained inclusion body was about 5 % in the weight ratio with the wet cells. This roughly purified fused protein was dissolved in a 8 M urea solution and purified by reverse phase HPLC. The protein eluted at an acetonitrile concentration of about 47 % was the fused protein.

Example 14

Reaction of the cleavage of the fused protein with thrombin and the amino acid sequencing of the sequence D' of human osteocalcin

A 8 M urea solution of the fused protein obtained in Example 13 was 10 times diluted with $H_2O$ (the protein concentration becomes about 1.0 mg/ml), and bovine thrombin (produced by Mochida Pharmaceutical Co., Ltd.) was added to portions of the dilution so that the final concentration became 0, 1, 3, 10 and 30 units/ml, and the mixtures were held at 37°C for 2 hours. The results of SDS-PAGE of the reaction solutions are shown in Figure 26. It is seen that the fused protein (molecular weight: about 26,000) was cleaved with the thrombin and the band (arrow) of the objective human osteocalcin sequence D' (molecular weight: about 5,500) was formed.

Further, the thrombin-untreated dilution and the thrombin-treated dilution (2 hours incubation at 30 units/ml and at 37°C) were subjected to reverse phase HPLC; the eluate of the main peak (acetonitrile concentration: about 42 %) newly formed after the thrombin treatment was taken, lyophilized, dissolved in trifluoroacetic acid and adsorbed on a polybulene treated filter according to the manual of Applied Biosystems Inc., the PTH-amino acids were cut off from the N-terminus using a protein sequencer (produced by Applied Biosystems Inc., 470A); and analysis was carried out using a PTH analyzer (produced by Applied Biosystems Inc., 120A), whereby the amino acid sequence up to 5 residues from the N-terminus was determined. As a results, the amino acid sequence accorded with the amino acid sequence up to 5 residues from the N-terminus of sequence D' (Tyr-Leu-Tyr-Glu-Trp).

Further, the molecular weight determined by SDS-PAGE accorded with the value estimated from sequence D', too.

From these results, it could be ascertained that human osteocalcin sequence D' was correctly cut out from the fused protein by the thrombin treatment.

References;

(1) M. DEMETZ et al; FEBS LETTERS, Vol 123, No. 2, pp215 218 (1981)
(2) Donald O. Mack et al; J. Bio. Chem., Vol. 251, No. 11, pp3269-3276, (1976)
(3) S. Nishikawa et al; Protein Engineering, Vol. 1, No. 6, pp487-492, (1987)
(4) Brian R. Hubbard et al P.N.A.S., Vol. 86, pp6893-6897 (1989)
(5) Magda M.W. Ulrich et al J. Bio. Chem., Vol. 263, No. 20, pp9697-9702, (1988)
(6) Thomas J. Gardella et al J. Bio. Chem., Vol. 265, No. 26, pp15854-15859 (1990)
(7) Berry A. M. Soute et al Thrombosis and Haemostasis, 57(1) 77-81 (1987)
(8) Alex Cheung et al, Archives of Biochemistry and Biophysics, Vol. 274, No. 2, pp574-581 (1989)
(9) A. J. Celeste et al, The EMBO Journal, Vol. 5, No. 8 pp1885-1890 (1986)
(10) M. Ikeda et al, P.N.A.S., Vol. 81 pp5956-5960 (1984)
(11) WO 87/04719
(12) WO 91/01372
(13) U. S. Patent Nos. 4,366,246, 4,425,437, 4,431,739, 4,563,424 and 4,812,554
(14) Japanese Laid-Open Patent Publication 234584/85

SEQUENCE LISTING

(1) INFORMATION FOR SEQ ID NO: 1

(i) Sequence characteristics:

(A) Length : 147

(B) Type: nucleic acid

(C) Strandness: double

(D) Topology: linear

(ii) Molecule type: other nucleic acid (synthetic DNA)

```
                         10                  20                  30
TACCTGTATCAGTGGCTGGGCGCTCCGGTT
(TyrLeuTyrGlnTrpLeuGlyAlaProVal
                         40                  50                  60
CCATACCCGGACCCTCTCGAGCCGCGCCGT
ProTyrProAspProLeuGluProArgArg
                         70                  80                  90
GAGGTTTGTGAACTGAACCCGGACTGCGAT
GluValCysGluLeuAsnProAspCysAsp
                        100                 110                 120
GAGCTGGCTGATCACATCGGTTTTCAGGAA
GluLeuAlaAspHisIleGlyPheGlnGlu
                        130                 140
GCATACCGTCGCTTCTACGGTCCGGTA
AlaTyrArgArgPheTyrGlyProVal)
```

(2) INFORMATION FOR SEQ ID NO: 2

(i) Sequence characteristics:

(A) Length: 66

(B) Type: nucleic acid

(C) Strandness: double

(D) Topology: linear

(ii) Molecule type: other nucleic acid (synthetic DNA)

(iii) Feature:

(A) Name/key: part of propeptide

(B) Location: 1 .... 66

(C) Identification method:

by experiment

```
                 10                  20                  30
AAACCGTCTGGCGCCGAATCTTCCAAAGCATTC
(LysProSerGlyAlaGluSerSerLysAlaPhe

           40                  50                  60
GTTTCTAAGCAGGAGGGATCCGAAGTTGTAAAA
ValSerLysGlnGluGlySerGluValValLys)
```

(3) INFORMATION FOR SEQ ID NO: 3

(i) Sequence characteristics:

(A) Length: 12

(B) Type: nucleic acid

(C) Strandness: double

(D) Topology: linear

(ii) Molecule type: other nucleic acid (synthetic DNA)

(iii) Feature:

(A) Name/key: thrombin cleavage site

(B) Location: 1 .... 12

(C) Identification method:

by experiment

10
CTGGTTCCGCGG
(LeuValProArg)

(4) INFORMATION FOR SEQ ID NO: 4

(i) Sequence characteristics:

(A) Length: 66

(B) Type: nucleic acid

(C) Strandness: double

(D) Topology: linear

(ii) Molecule type: other nucleic acid (synthetic DNA)

(iii) Feature:

(A) Name/key: sig peptide

(B) Location: 1 .... 66

(C) Identification method:

by experiment

```
          10                  20                  30
CGTGCTCTGACCCTGCTAGCCCTGCTGGCACTG
(ArgAlaLeuThrLeuLeuAlaLeuLeuAlaLeu

       40                  50                  60
GCTGCACTATGCATCGCAGGTCAGGCAGGTGCT
AlaAlaLeuCysIleAlaGlyGlnAlaGlyAla)
```

(5) INFORMATION FOR SEQ ID NO: 5

(i) Sequence characteristics:

(A) Length: 98

(B) Type: amino acid

(C) Topology: linear

(ii) Molecule type: protein

(iii) Immediate source:

(A) Library: Human Genomic Library

(B) Clone:

(iv) Position in genome:

(A) Chromosome/segment: Chromosome 1

(B) Map position:

(C) Units:

(v) FEATURE:

(A) Name/key:

(B) Location: 1 ... 23 S sig. peptide

24 ... 49 S pro. peptide

56 ... 98 S met. peptide

(C) Identification method:

MetArgAlaLeuThrLeuLeuAlaLeuLeu

AlaLeuAlaAlaLeuCysIleAlaGlyGln

AlaGlyAlaLysProSerGlyAlaGluSer

SerLysAlaPheValSerLysGlnGluGly

SerGluValValLysArgProArgArgTyr

LeuTyrGlnTrpLeuGlyAlaProValPro

TyrProAspProLeuGluProArgArgGlu

ValCysGluLeuAsnProAspCysAspGlu

LeuAlaAspHisIleGlyPheGlnGluAla

TyrArgArgPheTyrGlyProVal

(6) INFORMATION FOR SEQ ID NO: 6

(i) Sequence characteristics:

(A) Length: 310

(B) Type: nucleic acid

(C) Strandness: double

(D) Topolohy: linear

(ii) Molecule type: other nucleic acid (synthetic DNA)

(iii) Feature:

(A) Name/key:

(B) Location: 11 ... 76 E sig. peptide

77... 142 E pro. peptide

143... 154 E cleavage-site

155... 300 E mat. peptide

(C) Identification method:

by experiment

(11) 10 20 30 40 50 (9) 60

GAATTCCGAT CGTGCTCTGA CCCTGCTAGC CCTGCTGGCA CTGGCTGCAC TATGCATCGC

CTTAAGGCTA GCACGAGACT GGGACGATCG GGACGACCGT GACCGACGTG ATACGTAGCG

EcoRI (12) PvuI NheI BglI AvaIII (10)

70 80 90 100 110 (7) 120

AGGTCAGGCA GGTGCTAAAC CGTCTGGCGC CGAATCTTCC AAAGCATTCG TTTCTAAGCA

TCCAGTCCGT CCACGATTTG GCAGACCGCG GCTTAGAAGG TTTCGTAAGC AAAGATTCGT

BbeI (8)

130 140 150 160 (5) 170 180

GGAGGGATCC GAAGTTGTAA AACTGGTTCC GCGGTACCTG TATCAGTGGC TGGGCGCTCC

CCTCCCTAGG CTTCAACATT TTGACCAAGG CGCCATGGAC ATAGTCACCG ACCCGCGAGG

BamHI KpnI (6)

190 200 210 (1) 220 230 240

GGTTCCATAC CCGGACCCTC TCGAGCCGCG CCGTGAGGTT TGTGAACTGA ACCCGGACTG

CCAAGGTATG GGCCTGGGAG AGCTCGGCGC GGCACTCCAA ACACTTGACT TGGGCCTGAC

XhoI (2)

250 260 (3) 270 280 290 300

CGATGAGCTG GCTGATCACA TCGGTTTTCA GGAAGCATAC CGTCGCTTCT ACGGTCCGGT

GCTACTCGAC CGACTAGTGT AGCCAAAAGT CCTTCGTATG GCAGCGAAGA TGCCAGGCCA

BclI (4)

310

ATAATCTAGA

TATTAGATCT

XbaI

(7) INFORMATION FOR SEQ ID NO: 7

(i) Sequence characteristics:

(A) Lenght: 310

(B) Type: nucleic acid

(C) Strandness: double

(D) Topology: linear

(ii) Molecule type: other nucleic acid (synthetic DNA)

(iii) Feature:

(A) Name/key:

(B) Location: 11 ... 76 E sig. peptide

77 ... 142 E pro. peptide

143 ... 154 E cleavage-site

155 ... 300 E mat. peptide

(C) Identification method:

```
        10        20        30        40        50        60
GAATTCCGATCGTGCTCTGACCCTGCTAGCCCTGCTGGCACTGGCTGCACTATGCATCGC
          ArgAlaLeuThrLeuLeuAlaLeuLeuAlaLeuAlaAlaLeuCysIleAla

          |<------------------ A sequence ----------------------

        70        80        90       100       110       120
AGGTCAGGCAGGTGCTAAACCGTCTGGCGCCGAATCTTCCAAAGCATTCGTTTCTAAGCA
 GlyGlnAlaGlyAlaLysProSerGlyAlaGluSerSerLysAlaPheValSerLysGln

------------->|<--------------------- B sequence ---------------

       130       140       150       160       170       180
GGAGGGATCCGAAGTTGTAAAACTGGTTCCGCGGTACCTGTATCAGTGGCTGGGCGCTCC
 GluGlySerGluValValLysLeuValProArgTyrLeuTyrGlnTrpLeuGlyAlaPro

----------------------->|<-C sequence->|<-----------------------

       190       200       210       220       230       240
GGTTCCATACCCGGACCCTCTCGAGCCGCGCCGTGAGGTTTGTGAACTGAACCCGGACTG
 ValProTyrProAspProLeuGluProArgArgGluValCysGluLeuAsnProAspCys

-------------------------------------------- D sequence --------

       250       260       270       280       290       300
CGATGAGCTGGCTGATCACATCGGTTTTCAGGAAGCATACCGTCGCTTCTACGGTCCGGT
 AspGluLeuAlaAspHisIleGlyPheGlnGluAlaTyrArgArgPheTyrGlyProVal

----------------------------------------------------------------

       310
ATAATCTAGA
 ***

->|--------
```

(8) INFORMATION FOR SEQ ID NO: 8

(i) Sequence characteristics:

(A) Length: 21

(B) Type: amino acid

(C) Topology: linear

(ii) Molecule type: peptide

| 1 | 2 | 3 | 4 | 5 | 6 | 7 | 8 | 9 | 10 |
|---|---|---|---|---|---|---|---|---|---|
| Tyr - | Leu - | Tyr - | Gln - | Trp - | Leu - | Gly - | Ala - | Pro - | Val - |
| 11 | 12 | 13 | 14 | 15 | 16 | 17 | 18 | 19 | 20 |
| Pro - | Tyr - | Pro - | Asp - | Pro - | Leu - | Glu - | Pro - | Arg - | Arg |
| 21 | | | | | | | | | |
| Cys | | | | | | | | | |

(9) INFORMATION FOR SEQ ID NO: 9

(i) Sequence characteristics:

(A) Length: 7

(B) Type: amino acid

(C) Topology: linear

(ii) Molecule type: peptide

| 1 | 2 | 3 | 4 | 5 | 6 | 7 |
|---|---|---|---|---|---|---|
| Arg - | Arg - | Phe - | Tyr - | Gly - | Pro - | Val |

(10) INFORMATION FOR SEQ ID NO: 10

(i) Sequence characteristics:

(A) Length: 15

(B) Type: amino acid

(C) Topology: linear

(ii) Molecule type: peptide

| 1 | 2 | 3 | 4 | 5 | 6 | 7 | 8 | 9 | 10 |
|---|---|---|---|---|---|---|---|---|---|
| Cys - | Ile - | Gly - | Phe - | Gln - | Glu - | Ala - | Tyr - | Arg - | Arg |

| 11 | 12 | 13 | 14 | 15 |
|---|---|---|---|---|
| Phe - | Tyr - | Gly - | Pro - | Val |

(11) INFORMATION FOR SEQ ID NO: 11

(i) Sequence characteristics:

(A) Length: 84

(B) Type: nucleic acid

(C) Strandness: double

(D) Topology: linear

(ii) Molecule type: other nucleic acid (synthetic DNA)

(iii) Immediate source:

(A) Library:

(B) Clone:

(iv) Position in genome:

(A) Chromosome/segment:

(B) Map position:

(C) Units:

(v) Feature:

(A) Name/key: part of sig. peptide

(B) Location 11 ... 38

(C) Identification method:

by experiment

```
         10            20   (13)     30          40
GAATTCCTTT AAACGTGCTC TGACCCTGCT AGCCCTGCTG GC

CTTAAGGAAA TTTGCACGAG ACTGGGACGA TCGGGACGAC CG
  EcoRI     DraI      (14)        NheI    BglI
```

## Claims

**1.** A DNA sequence for the expression of human osteocalcin represented by the following formula [I]

A-B-C-D [I]

wherein,

- D represents a DNA sequence encoding human osteocalcin or a protein physiologically equal thereto (sequence D),
- C represents a DNA sequence encoding a cleavage sequence peptide (sequence C),
- B represents B-1 and/or B-2 wherein B-1 represents DNA sequence encoding an additional polypeptide for expressing human osteocalcin in a large amount in host cells (sequence B-1) and B-2 represents a DNA sequence encoding a polypeptide recognized by an enzyme having an ability to convert all or part of the Glu residues in the amino acid sequence of human osteocalcin to Gla residues (hereinafter referred to as an enzyme capable of Glu to Gla conversion on human osteocalcin) (sequence B-2), and

A represents a DNA sequence which may optionally be present and encodes a signal peptide (sequence A).

**2.** The DNA sequence for the expression of human osteocalcin of claim 1 wherein sequence D is the DNA sequence shown in Figure 1.

**3.** The DNA sequence for the expression of human osteocalcin of Claim 1 wherein sequence C is the DNA sequence shown in Figure 3.

**4.** The DNA sequence for the expression of human osteocalcin of claim 1 wherein B consists of B-1 and B-2 (sequence B-1 and sequence B-2).

**5.** The DNA sequence for the expression of human osteocalcin of claim 1 wherein sequence B-2 is the DNA sequence shown in Figure 2.

**6.** The DNA sequence for the expression of human osteocalcin of claim 1 wherein sequence B-1 is a DNA sequence encoding human growth hormone.

**7.** A vector for the expression of human osteocalcin which contains the DNA sequence for the expression of human osteocalcin of claim 1 and can express it in host cells.

**8.** The vector for the expression of human osteocalcin of claim 7 which contains the DNA sequence for the expression of human osteocalcin of claim 1 and a DNA sequence having a function regulating the expression of human osteocalcin (expression-regulating sequence).

**9.** The vector for the expression of human osteocalcin of claim 8 wherein the expression-regulating sequence is an expression-regulating sequence capable of functioning in Escherichia coli.

**10.** The vector for the expression of human osteocalcin of claim 7 which is a plasmid capable of self-replication in Escherichia coli.

**11.** Recombinant host cells wherein the transformation was carried out with the vector for the expression of human osteocalcin of claim 7.

**12.** The recombinant host cells of claim 11 wherein the host cells are Escherichia coli.

**13.** A process for preparing human osteocalcin or a protein physiologically equal thereto which comprises culturing the recombinant host cells of claim 11 to form in the culture broth a polypeptide-containing protein represented by the following formula [II]

B'-C'-D' [II]

wherein

D' represents human osteocalcin or the protein physiologically equal thereto,

C' represents a cleavage sequence peptide, and

B' represents B'-1 and/or B'-2 wherein B'-1 represents a polypeptide recognized by an enzyme capable of Glu to Gla conversion on human osteocalcin and B'-2 represents an additional polypeptide for expressing human osteocalcin in a large amount in the host cells;

cleaving this protein; and isolating human osteocalcin or the protein physiologically equal thereto (D').

**14.** A process for preparing human osteocalcin or a protein physiologically equal thereto which comprises culturing the recombinant host cells of claim 11 to form in the culture broth a polypeptide-containing protein represented by the following formula [II]

B'-C'-D'     [II]

wherein

D' represents human osteocalcin or the protein physiologically equal thereto,

C' represents a cleavage sequence peptide, and

B' represents B'-1 and/or B'-2 wherein B'-1 represents a polypeptide recognized by an enzyme capable of Glu to Gla conversion on human osteocalcin and B'-2 represents an additional polypeptide for expressing human osteocalcin in a large amount in the host cells;

converting all or part of the Glu residues in the D' protein in the protein to Gla residues; cleaving this protein; and isolating human osteocalcin or the protein physiologically equal thereto.

**15.** Human osteocalcin or a protein physiologically equal thereto obtained by the preparation process of any of claims 13 and 14.

**16.** The recombinant Escherichia coli K-12 strain XLI-Blue$F^-$ [pKOC 28] deposited with Fermentation Research Institute, Agency of Industrial Science and Technology under the accession number of BP-2950.

**17.** The recombinant Escherichia coli K-12 strain HBl0l [pGH-POCI] deposited with Fermentation Research Institute, Agency of Industrial Science and Technology under the accession number of BP-3171.

# Fig. 1

( sequence D )

```
         10                  20                  30
TACCTGTATCAGTGGCTGGGCGCTCCGGTT
(TyrLeuTyrGlnTrpLeuGlyAlaProVal

         40                  50                  60
CCATACCCGGACCCTCTCGAGCCGCGCCGT
ProTyrProAspProLeuGluProArgArg

         70                  80                  90
GAGGTTTGTGAACTGAACCCGGACTGCGAT
GluValCysGluLeuAsnProAspCysAsp

        100                 110                 120
GAGCTGGCTGATCACATCGGTTTTCAGGAA
GluLeuAlaAspHisIleGlyPheGlnGlu

        130                 140
GCATACCGTCGCTTCTACGGTCCGGTA
AlaTyrArgArgPheTyrGlyProVal)
```

# Fig. 2

( sequence B )

```
         10                  20                  30
AAACCGTCTGGCGCCGAATCTTCCAAAGCATTC
(LysProSerGlyAlaGluSerSerLysAlaPhe

         40                  50                  60
GTTTCTAAGCAGGAGGGATCCGAAGTTGTAAAA
ValSerLysGlnGluGlySerGluValValLys)
```

# Fig. 3

(sequence C)

```
           10
CTGGTTCCGCGG
(LeuValProArg)
```

# Fig. 4

(sequence A)

```
          10        20        30
CGTGCTCTGACCCTGCTAGCCCTGCTGGCACTG
(ArgAlaLeuThrLeuLeuAlaLeuLeuAlaLeu

       40        50        60
GCTGCACTATGCATCGCAGGTCAGGCAGGTGCT
AlaAlaLeuCysIleAlaGlyGlnAlaGlyAla)
```

# Fig. 5

MetArgAlaLeuThrLeuLeuAlaLeuLeu

AlaLeuAlaAlaLeuCysIleAlaGlyGln

prepeptide part

AlaGlyAlaLysProSerGlyAlaGluSer

SerLysAlaPheValSerLysGlnGluGly

propeptide part

SerGluValValLysArgProArgArgTyr

LeuTyrGlnTrpLeuGlyAlaProValPro

TyrProAspProLeuGluProArgArgGlu

mature part

ValCysGluLeuAsnProAspCysAspGlu

LeuAlaAspHisIleGlyPheGlnGluAla

TyrArgArgPheTyrGlyProVal

# Fig. 6

⑪ 10 20 30 40 50 ⑨ 60

GAATTCCGAT CGTGCTCTGA CCCTGCTAGC CCTGCTGGCA CTGGCTGCAC TATGCATCGC

CTTAAGGCTA GCACGAGACT GGGACGATCG GGACGACCGT GACCGACGTG ATACGTAGCG

EcoRI ⑫ PvuI NheI BglI AvaIII ⑩

70 80 90 100 110 ⑦ 120

AGGTCAGGCA GGTGCTAAAC CGTCTGGCGC CGAATCTTCC AAAGCATTCG TTTCTAAGCA

TCCAGTCCGT CCACGATTTG GCAGACCGCG GCTTAGAAGG TTTCGTAAGC AAAGATTCGT

BbeI ⑧

130 140 150 160 ⑤ 170 180

GGAGGATCC GAAGTTGTAA AACTGGTTCC GCGGTACCTG TATCAGTGGC TGGGCGCTCC

CCTCCCTAGG CTTCAACATT TTGACCAAGG CGCCATGGAC ATAGTCACCG ACCCGCGAGG

BamHI KpnI ⑥

190 200 210 ① 220 230 240

GGTTCCATAC CCGGACCCTC TCGAGCCGCG CCGTGAGGTT TGTGAACTGA ACCCGGACTG

CCAAGGTATG GGCCTGGGAG AGCTCGGCGC GGCACTCCAA ACACTTGACT TGGGCCTGAC

XhoI ②

250 260 ③ 270 280 290 300

CGATGAGCTG GCTGATCACA TCGGTTTTCA GGAAGCATAC CGTCGCTTCT ACGGTCCGGT

GCTACTCGAC CGACTAGTGT AGCCAAAAGT CCTTCGTATG GCAGCGAAGA TGCCAGGCCA

BclI ④

310

ATAATCTAGA

TATTAGATCT

XbaI

The solid lines denote the boundaries of the synthesized DNA fragments and the dotted lines denote the cleavage sites with the restriction enzymes

# Fig. 7

```
         10        20        30        40        50        60
GAATTCCGATCGTGCTCTGACCCTGCTAGCCCTGCTGGCACTGGCTGCACTATGCATCGC
          ArgAlaLeuThrLeuLeuAlaLeuLeuAlaLeuAlaAlaLeuCysIleAla
                          sequence A

         70        80        90       100       110       120
AGGTCAGGCAGGTGCTAAACCGTCTGGCGCCGAATCTTCCAAAGCATTCGTTTCTAAGCA
 GlyGlnAlaGlyAlaLysProSerGlyAlaGluSerSerLysAlaPheValSerLysGln
                          sequence B

        130       140       150       160       170       180
GGAGGGATCCGAAGTTGTAAAACTGGTTCCGCGGTACCTGTATCAGTGGCTGGGCGCTCC
 GluGlySerGluValValLysLeuValProArgTyrLeuTyrGlnTrpLeuGlyAlaPro
                      sequence C

        190       200       210       220       230       240
GGTTCCATACCCGGACCCTCTCGAGCCGCGCCGTGAGGTTTGTGAACTGAACCCGGACTG
 ValProTyrProAspProLeuGluProArgArgGluValCysGluLeuAsnProAspCys
                          sequence D

        250       260       270       280       290       300       310
CGATGAGCTGGCTGATCACATCGGTTTTCAGGAAGCATACCGTCGCTTCTACGGTCCGGTATAATCTAGA
 AspGluLeuAlaAspHisIleGlyPheGlnGluAlaTyrArgArgPheTyrGlyProVal***
```

Fig. 8

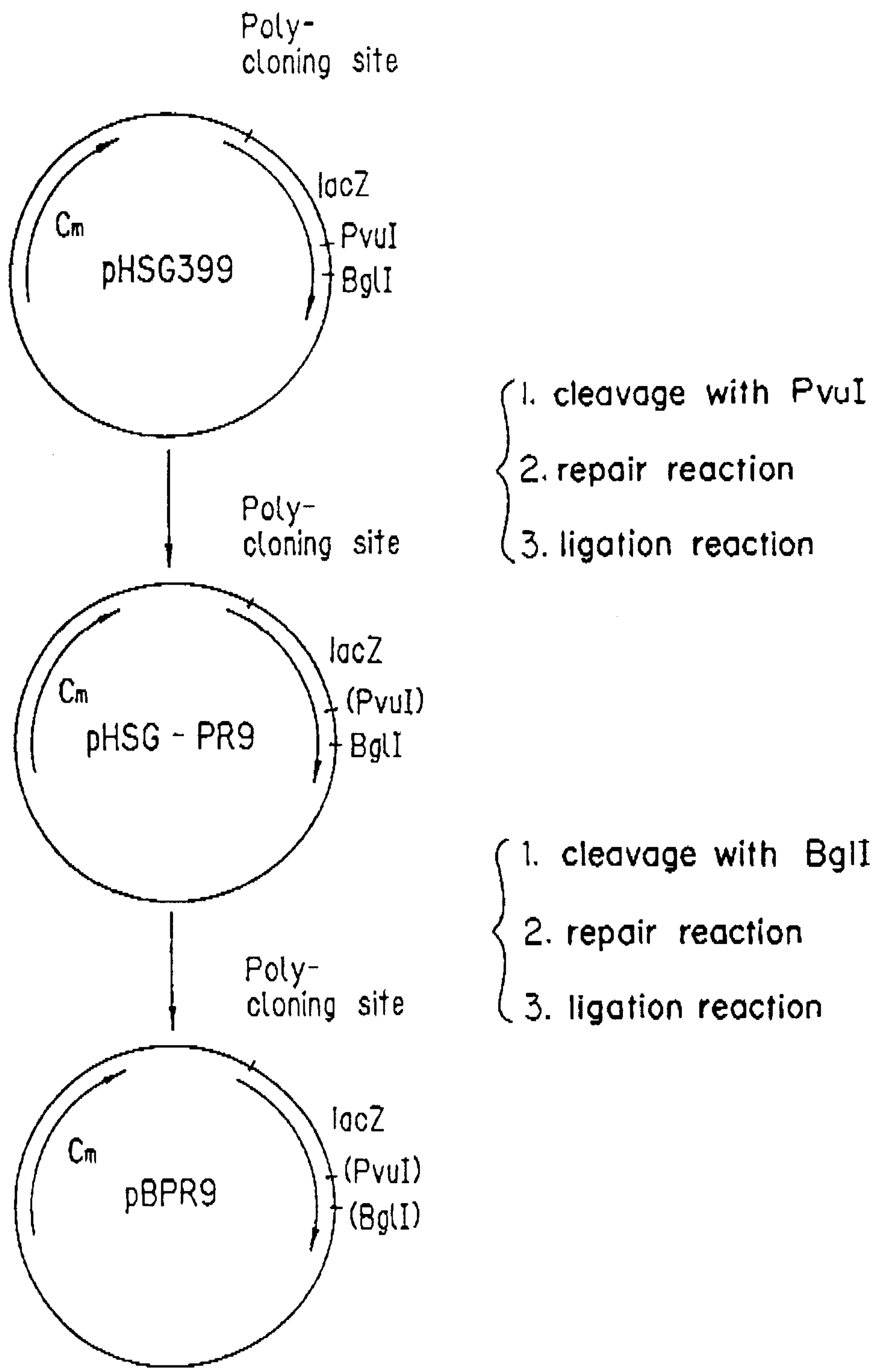
Poly-
cloning site
lacZ
Cm
pHSG399
PvuI
BglI
1. cleavage with PvuI
2. repair reaction
3. ligation reaction
Poly-
cloning site
lacZ
Cm
pHSG - PR9
(PvuI)
BglI
1. cleavage with BglI
2. repair reaction
3. ligation reaction
Poly-
cloning site
lacZ
Cm
pBPR9
(PvuI)
(BglI)

# Fig. 9

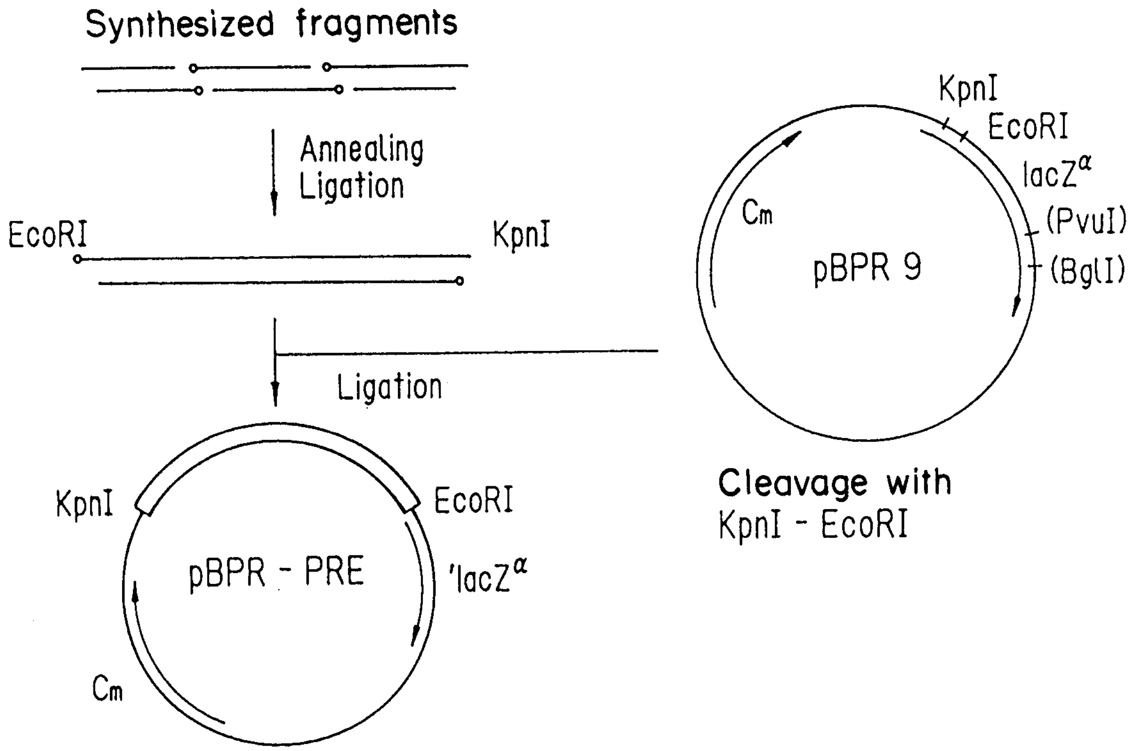
Synthesized fragments
Annealing
Ligation
EcoRI
KpnI
Ligation
KpnI
EcoRI
pBPR - PRE
'lacZ$^{\alpha}$
Cm
KpnI
EcoRI
lacZ$^{\alpha}$
(PvuI)
(BglI)
Cm
pBPR 9
Cleavage with
KpnI - EcoRI

# Fig. 10

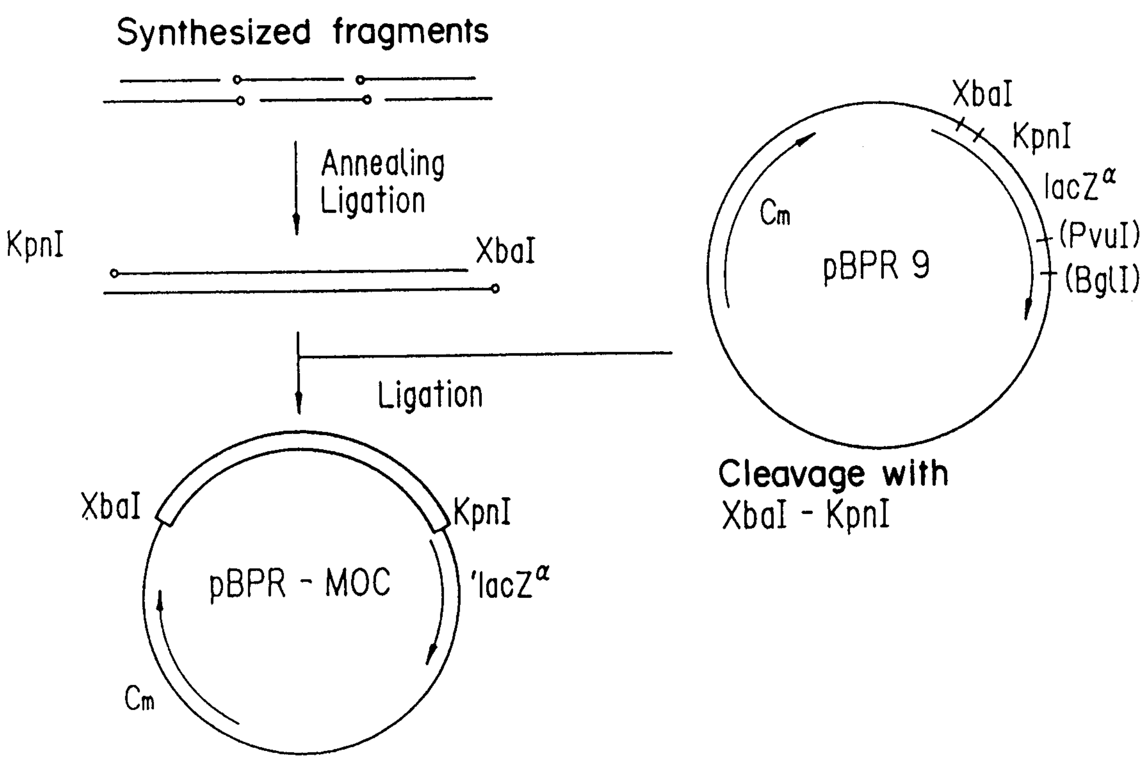
Synthesized fragments
Annealing
Ligation
KpnI
XbaI
Ligation
XbaI
KpnI
pBPR - MOC
'lacZ$^{\alpha}$
Cm
XbaI
KpnI
lacZ$^{\alpha}$
(PvuI)
(BglI)
Cm
pBPR 9
Cleavage with
XbaI - KpnI

Fig. 11

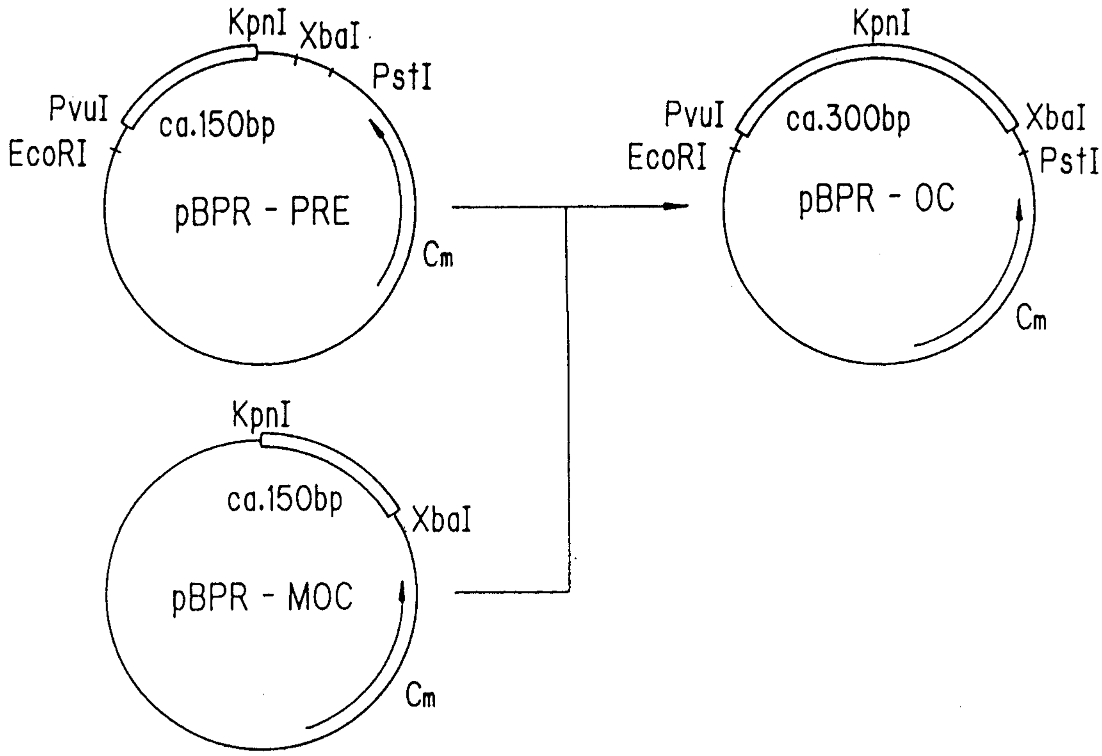
KpnI
XbaI
PstI
PvuI
EcoRI
ca.150bp
pBPR - PRE
Cm
KpnI
ca.150bp
XbaI
pBPR - MOC
Cm
KpnI
PvuI
EcoRI
ca.300bp
XbaI
PstI
pBPR - OC
Cm

Fig. 12

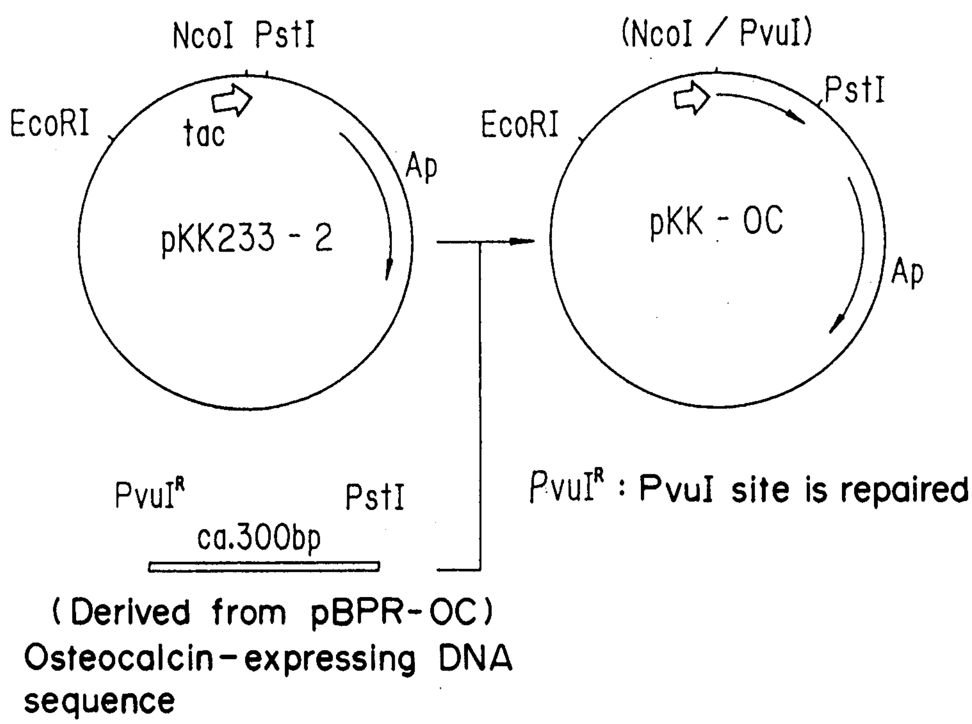
NcoI PstI
EcoRI
tac
Ap
pKK233 - 2
(NcoI / PvuI)
PstI
EcoRI
pKK - OC
Ap
PvuI$^R$
PstI
ca.300bp
(Derived from pBPR-OC)
Osteocalcin-expressing DNA
sequence
PvuI$^R$ : PvuI site is repaired

# Fig. 13

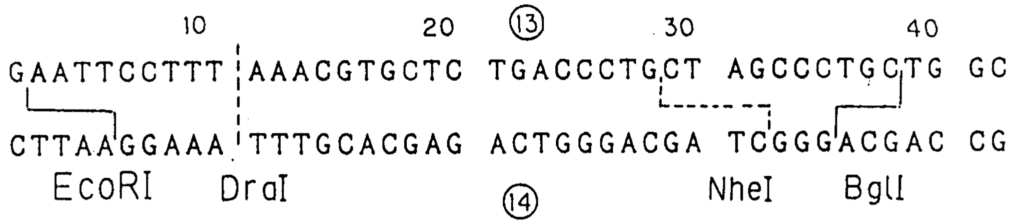


The solid lines denote both ends of the synthesized fragments and the dotted lines denote the cleavage sites with the restriction enzymes

# Fig. 14

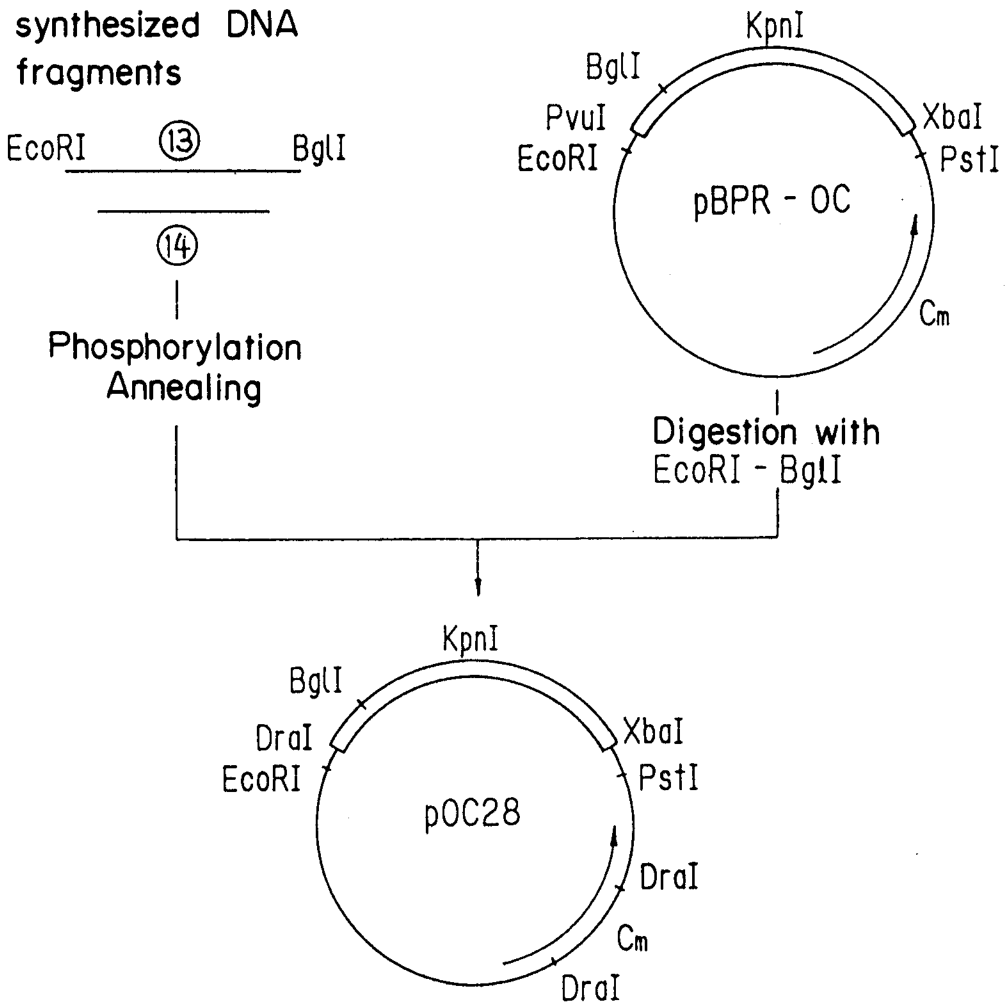

Fig. 15

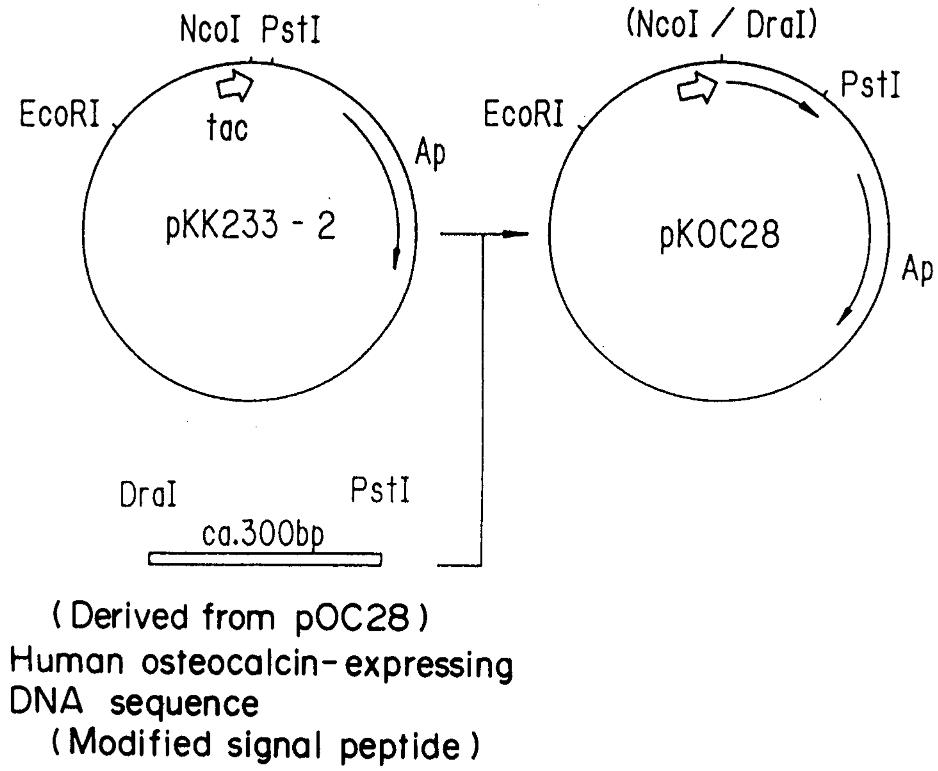
NcoI PstI
EcoRI
tac
Ap
pKK233 - 2
(NcoI / DraI)
PstI
EcoRI
pKOC28
Ap
DraI
PstI
ca.300bp
(Derived from pOC28)
Human osteocalcin-expressing
DNA sequence
(Modified signal peptide)

Fig. 17

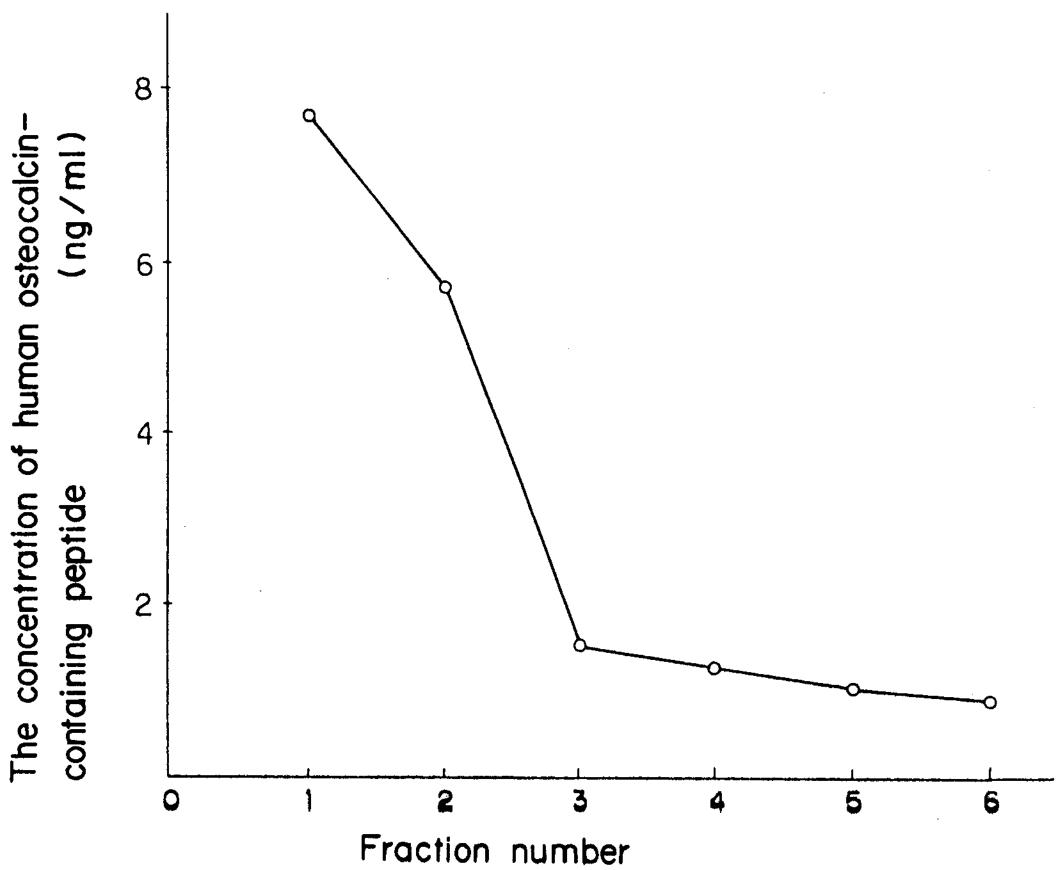
The concentration of human osteocalcin-containing peptide (ng/ml)
8
6
4
2
0
1
2
3
4
5
6
Fraction number

Fig. 16

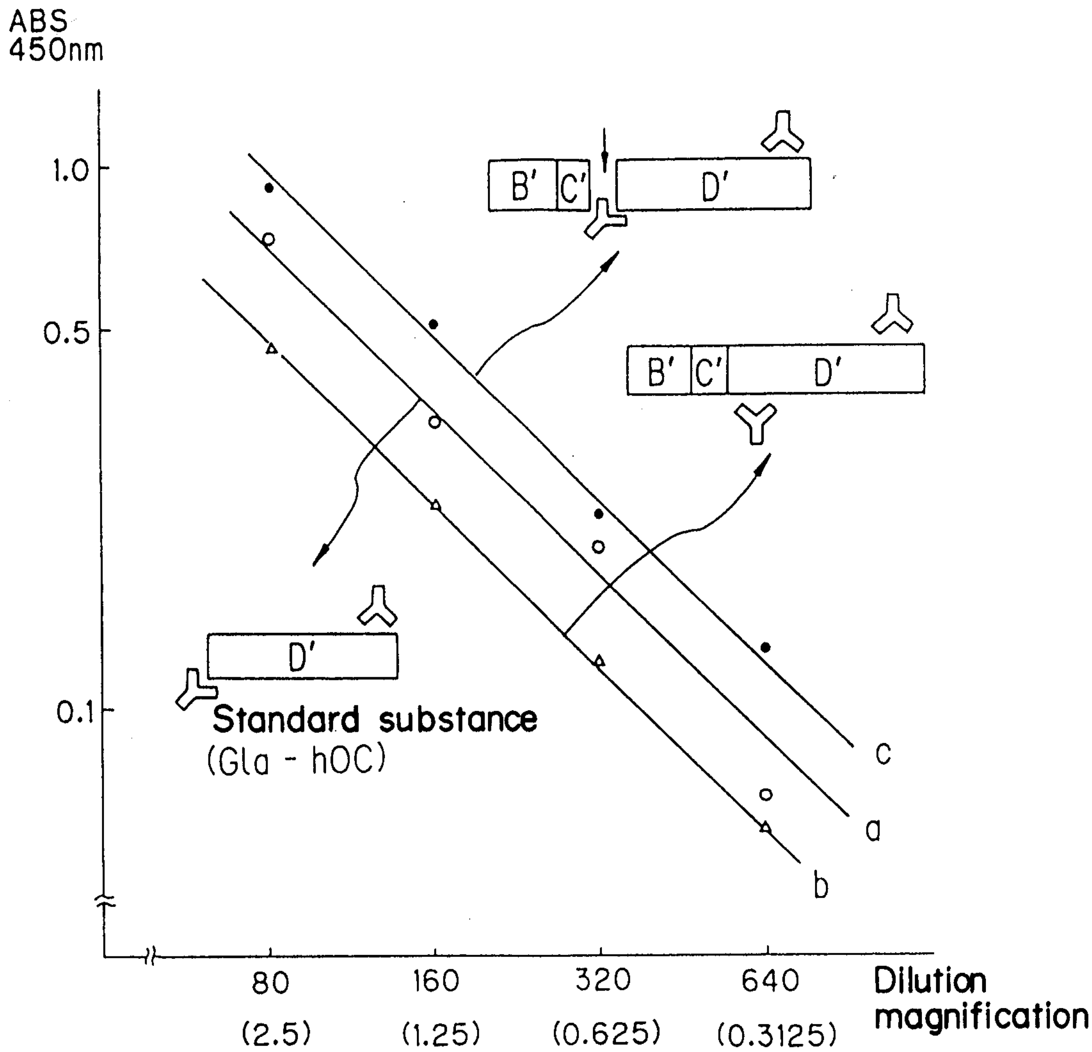


The parenthesis represents the concentration of the standard substance (ng/ml)

a : Standard curve (purified human osteocalcin (Gla-hOC) was used)
b : Periplasmic fraction
c : Thrombin-digested periplasmin fraction

# Fig. 18

(a)

H - Tyr[1] - Leu[2] - Tyr[3] - Gln[4] - Trp[5] - Leu[6] - Gly[7] - Ala[8] - Pro[9] - Val[10] - Pro[11] - Tyr[12] - Pro[13] - Asp[14] - Pro[15] - Leu[16] - Glu[17] - Pro[18] - Arg[19] - Arg[20] - Cys[21] - OH

(b)

Arg[1] - Arg[2] - Phe[3] - Tyr[4] - Gly[5] - Pro[6] - Val[7] - OH

(c)

H - Cys[1] - Ile[2] - Gly[3] - Phe[4] - Gln[5] - Glu[6] - Ala[7] - Tyr[8] - Arg[9] - Arg[10] - Phe[11] - Tyr[12] - Gly[13] - Pro[14] - Val[15] - OH

Fig. 19

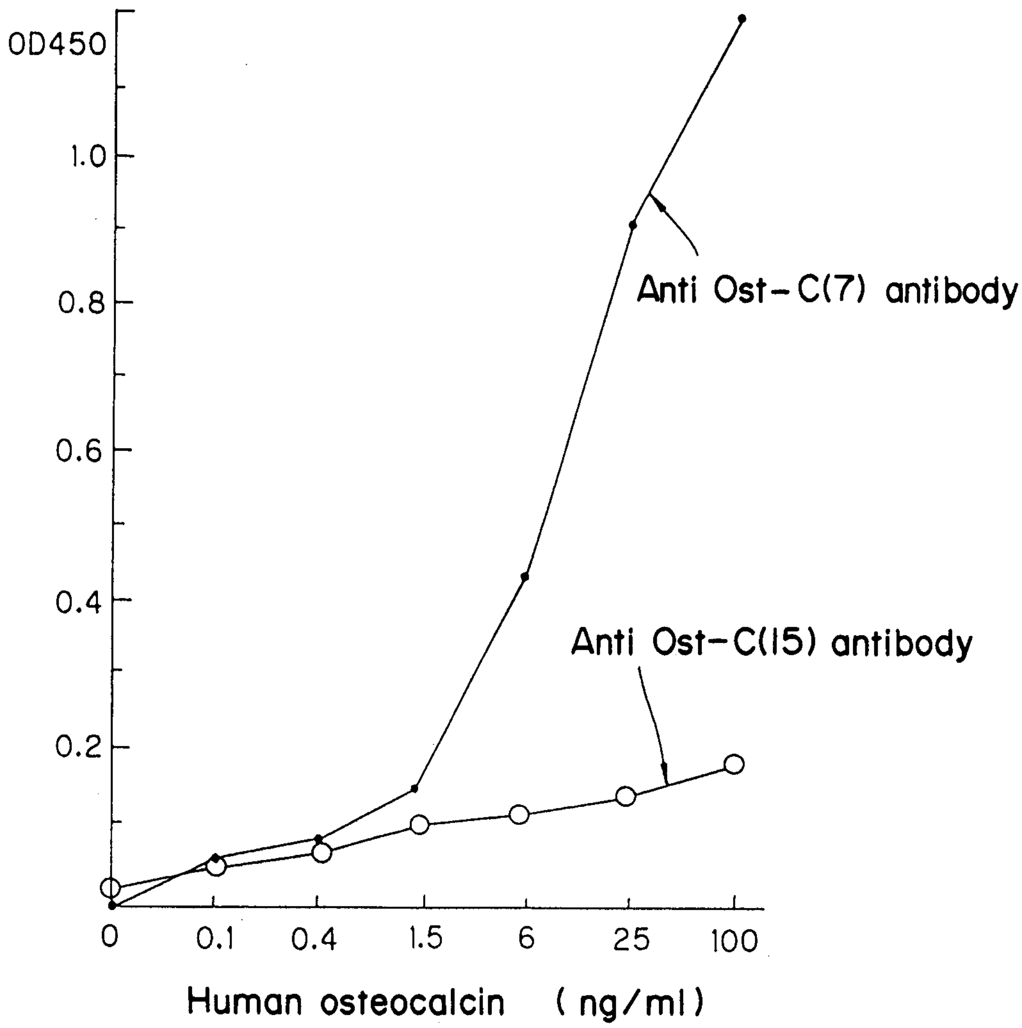
OD450
1.0
0.8
0.6
0.4
0.2
0
0.1
0.4
1.5
6
25
100
Human osteocalcin (ng/ml)
Anti Ost-C(7) antibody
Anti Ost-C(15) antibody

Fig. 20

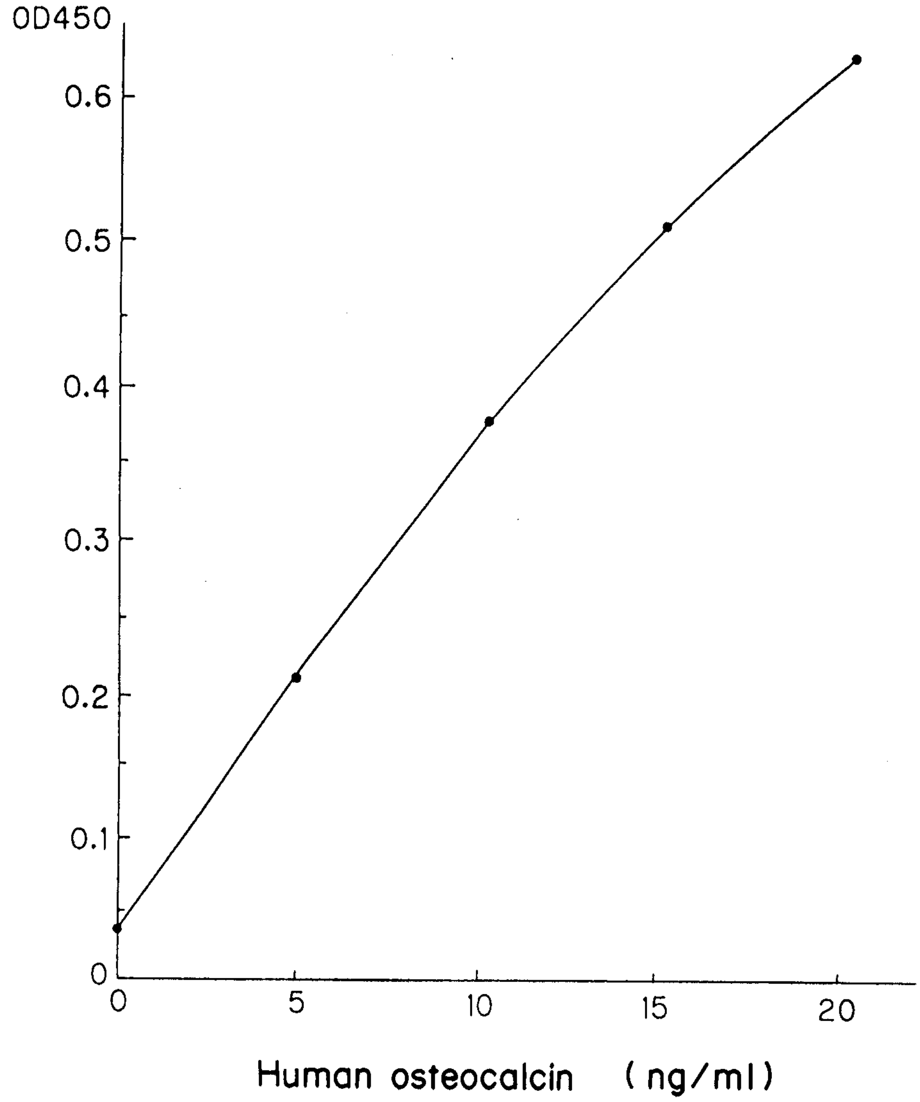

## Fig. 21

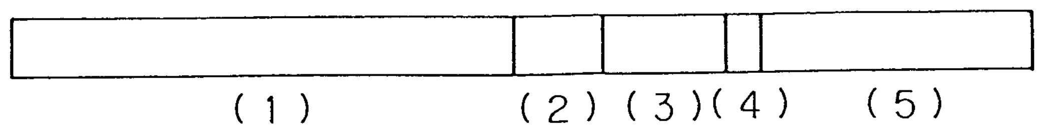


(1) . . . DNA sequence encoding human growth hormone (hGH)
(2) . . . DNA sequence encoding the FXa cleavage sequence
(3) . . . Sequence B
(4) . . . Sequence C
(5) . . . Sequence D

## Fig. 22

```
5'              10               20              30     3'
 GATC TTC ATC GAA GGT CGT AAA CCG TCT GGC GC   +strand
      AAG TAG CTT CCA GCA TTT GGC AGA C        -strand
      (Phe Ile Glu Gly Arg Lys Pro Ser Gly)
           └────────────────┘
           FXa cleavage sequence
```

Fig. 23

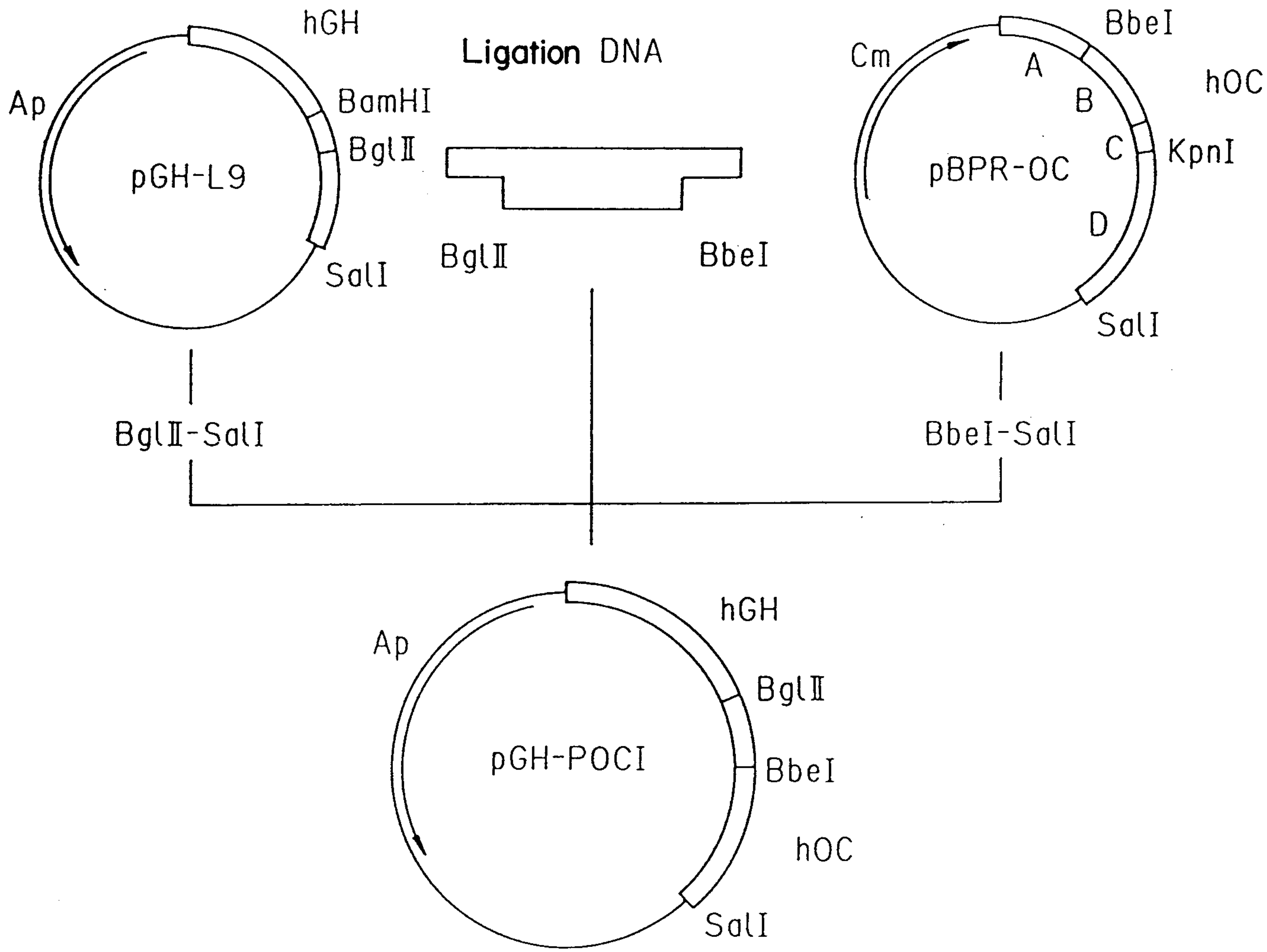
hGH
Ap
pGH-L9
BamHI
BglII
SalI
Ligation DNA
BglII
BbeI
Cm
pBPR-OC
BbeI
A
B
C
D
hOC
KpnI
SalI
BglII-SalI
BbeI-SalI
Ap
pGH-POCI
hGH
BglII
BbeI
hOC
SalI

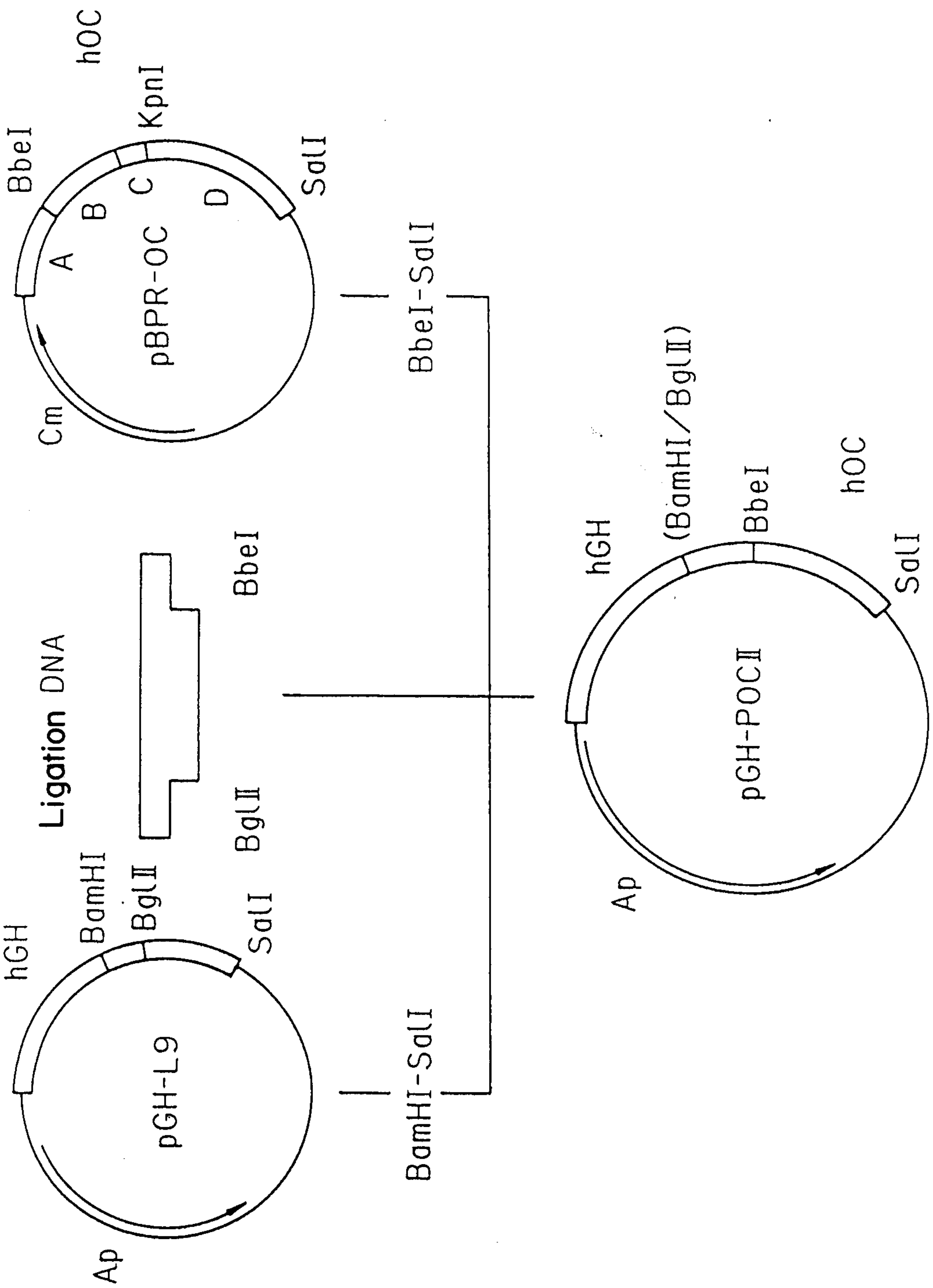
Ligation DNA
BglII
BbeI
hGH
BamHI
BglII
SalI
pGH-L9
Ap
BamHI-SalI
Cm
pBPR-OC
A
B
C
D
BbeI
KpnI
hOC
SalI
BbeI-SalI
hGH
(BamHI/BglII)
BbeI
hOC
SalI
pGH-POCII
Ap

Fig. 24

# Fig. 25

(A)

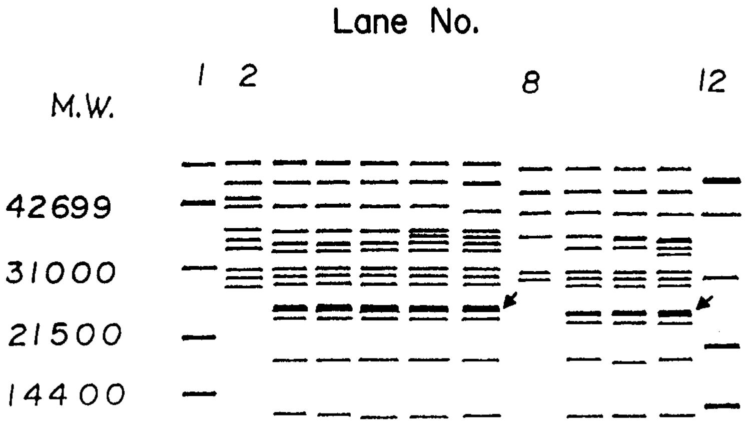


Lane No.
1 Marker
2~7
JM109[pGH-POCI]
After induction with IAA
0,2,3,4,5,6,hr
8~11
HB101[pGH-POCI]
After induction with IAA
0,2,3,4 hr
12 Marker

(B)

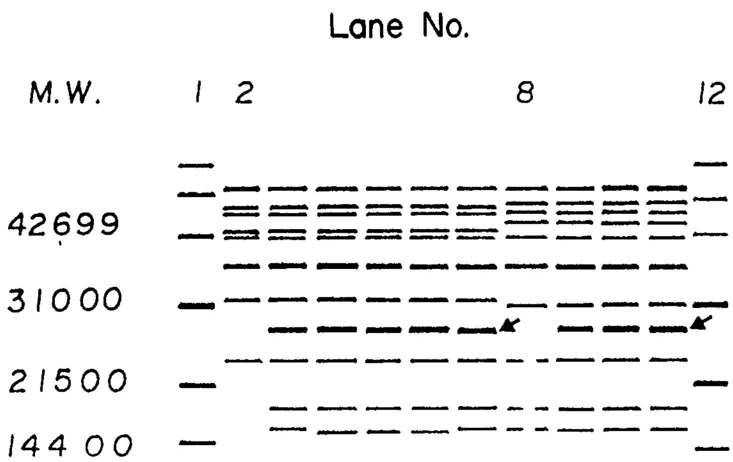


Lane No.
1 Marker
2~7
JM109[pGH-POCII]
After induction with IAA
0,2,3,4,5,6,hr
8~11
HB101[pGH-POCII]
After induction with IAA
0,2,3,4 hr
12 Marker

# Fig. 26

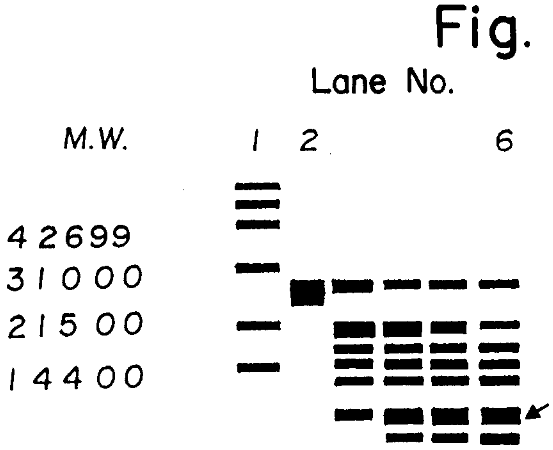


Lane No.
1 Marker
2~6
Bovine thrombin
0,1,3,10,30 unit/ml